(19) Europäisches Patentamt
European Patent Office
Office européen des brevets

(11) EP 2 578 579 A1

(12) **EUROPEAN PATENT APPLICATION**

(43) Date of publication:
**10.04.2013 Bulletin 2013/15**

(21) Application number: **11173782.1**

(22) Date of filing: **13.07.2011**

(51) Int Cl.:
***C07D 333/70*** $^{(2006.01)}$ ***A61K 31/381*** $^{(2006.01)}$
***A61P 33/06*** $^{(2006.01)}$

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.07.2010 IN DE16462010**

(71) Applicants:
- **National Institute of Immunology**
**New Delhi 110067 (IN)**
- **Indian Institute Of Science**
**Karnataka (IN)**

(72) Inventors:
- **Surolia, Avadhesha**
**110067 New Delhi (IN)**
- **Banerjee, Tanushree**
**110067 New Delhi (IN)**
- **Surolia, Namita**
**P.O. Bangalore - 560 064 (IN)**
- **Kapoor, Neha**
**110067 New Delhi (IN)**

(74) Representative: **Zacco Denmark A/S**
**Hans Bekkevolds Allé 7**
**2900 Hellerup (DK)**

Remarks:
The references to figures 10,13A,13B are deemed to be deleted (Rule 43 EPC 1973).

(54) **Benzothiophene carboxamide compounds, composition and applications thereof**

(57) The present invention provides benzothiophene carboxamide compounds of formula I, their polymorphs, stereoisomers, prodrugs, solvates, pharmaceutically acceptable salts and formulations thereof, which are useful as COX-2 inhibitors and PfENR inhibitors.

The invention further relates to pharmaceutical compositions containing such compounds and methods for their application as COX-2 inhibitors for treating inflammation and pain and PfENR inhibitors for use as anti-malarials.

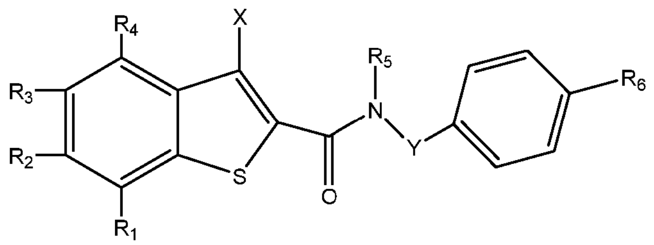

**Formula I**

EP 2 578 579 A1

## Description

### Field of the invention

The invention relates to benzothiophene carboxamide compounds of formula I, their polymorphs, stereoisomers, prodrugs, solvates, pharmaceutically acceptable salts and formulations thereof The compounds are useful as COX-2 inhibitors and PfENR inhibitors.

The invention further relates to pharmaceutical compositions containing such compounds and methods for their application as PfENR inhibitors for use as anti-malarials, and COX-2 inhibitor for treating inflammation and pain.

### Background

Non-steroidal anti-inflammatory drugs (NSAIDs) are among the most prescribed drugs that are used as treatment for pain and inflammation in acute or chronic conditions (Wolfe, M.M.et al; Gastrointestinal toxicity of non-steroidal anti-inflammatory drugs. N Engl J Med. 1999, 340, 1888-1899; Ong, C.K. et al. Combining paracetamol (acetaminophen) with nonsteroidal antiinflammatory drugs: a qualitative systematic review of analgesic efficacy for acute postoperative pain, Anesth Analg. 2010, 110, 1170-9). NSAIDs act by blocking the action of an enzyme known as cyclo-oxygenase (COX) which produces prostaglandins. Prostaglandins are important biological mediators of inflammation, originating from biotrasformation of arachidonic acid catalyzed by COX. It is known that the enzyme exists in two isoforms, one constitutive (COX-1) and the other inducible (COX-2). COX-1 is involved in several physiological functions. Conventional NSAID inhibit COX-1 and thus can have adverse effects on gastrointestinal tract. COX-2 is primarily an inducible enzyme that is not found in most tissues under normal conditions but is produced in response to pain and inflammation.

One of the major drawbacks of NSAIDs is that they are non-selective in nature towards two forms of cyclo-oxygenases namely COX-1 and COX-2. Both forms perform same function except that one is inducible and inflammatory (COX-2) while as other is constitutive (COX-1) and protective in nature. Most of the side effects of NSAIDs are attributed to COX-1 inhibition and reflected commonly as ulcers and bleeding. The frequency of side effects varies among NSAIDs but most common include vomiting, nausea, constipation, drowsiness and diarrhea, among others. To avoid the toxicity of NSAIDs due to the inhibition of coexisting COX-1, selective inhibitors of COX-2 have been investigated. The selective COX-2 inhibitors have anti-inflammatory action, pain-relieving action, and/or antipyretic action; with less side-effects such as bleeding in the gastrointestinal tract. COX-2 inhibitors may show anticancer activity and lower the induction of asthma in asthmatic patients who are sensitive to conventional NSAIDs.

US7375130 refers to certain benzofuran and benzothiophene compounds having antidiabetic properties.

US7351735 refers to certain benzofuran and benzothiophene derivatives useful in the treatment of hyperproliferative disorders.

US7148240, US6949583 and US6946483 refer to certain aminoalkoxybenzoyl-benzofuran or benzothiophene derivatives, method for preparing same and composition containing same.

US4663347, US4745127, US4822803, US4933351 and US4621091 refer to certain benzofuran-2-carboxylic acid derivatives as 5-lipoygenase inhibitors.

US4548948 discloses certain benzothiophene and benzofuran derivatives as anti-inflammatory and analgesic agent. Also, US6433005 discloses certain benzofuran and benzothiophene derivatives as anti inflammatory agents.

There is further a need for such heterocyclic compounds that are more potent in treatment and management of pain and inflammation.

Current insights on the *Plasmodium's* metabolome have uncovered different targets for the development of novel anti-malarials. Fatty acids biosysnthesis enzymes are one of the important targets. Tremendous proliferative potential of *Plasmodium* makes it essentially dependent on abundant supply of fatty acids. Fatty acids are required for membrane synthesis, lipid biogenesis and glycosylphosphatidylinositol (GPI) anchors of its transmembrane proteins. In the case of *Plasmodium, de novo* synthesis of fatty acids occurs by Type II Fatty Acid Synthase (FAS) which is fundamentally different from Type I Fatty Acid Synthase system of humans (Smith, S. et al, Structural and functional organization of the animal fatty acid synthase. Prog. Lipid Res. 2003, 42, 289-317; Ralph, S. A. et al., Metabolic maps and functions of the Plasmodium falciparum apicoplast. Nat. ReV. Microbiol. 2004, 2, 23-216; Rock, C. O. et al, Escherichia coli as a model for the regulation of dissociable (type II) fatty acid biosynthesis. Biochim. Biophys. Acta 1996, 132, 1-16; Surolia, N. et al, Triclosan offers protection against blood-stages of malaria by inhibiting enoyl-ACP reductase of Plasmodium falciparum. Nat. Med. 2001, 7, 167-173; Heath, R. J.et al, Curr. Opin. InVestig. Drugs 2004, 5, 46-53). Using Type II FAS enzyme knock out parasite lines, it has recently been shown to be indispensable for liver-stage parasite development which further strengthens its significance in the parasite biology (Singh, A.P. et al, Triclosan inhibit the growth of the late liver-stage of Plasmodium. IUBMB life 2009, 61, 923-928; Vaughan, A. M. et al, Type II Fatty Acid Is Essential for Only Late Liver-stage Development. Cellular Microbiology, 2009, 11, 56-520).

In Type II FAS acetoacetyl-ACP enters the elongation cycle which involves four reactions: Decarboxylative

condensation to condense the growing acyl chain with malonyl-ACP catalysed by β-ketoacyl-ACP synthase (FabF), NADPH dependent reduction by β-ketoacyl-ACP reductase (FabG), dehydration is catalysed by β-hydroxyacyl-ACP dehydratase (FabA or FabZ) and NADH dependent reduction by enoyl-ACP reductase (FabI or PfENR). PfENR catalyses the rate determining step of the elongation cycle and therefore has emerged as an important drug target (Rock, C. O. et al; Escherichia coli as a model for the regulation of dissociable (type II) fatty acid biosynthesis. Biochim. Biophys. Acta 1996, 132, 1-16; Mahmoudi, N. et al; In vitro activities of 25 quinolones and fluoroquinolones against liver and blood-stage Plasmodium spp. Antimicrobial Agents and Chemotherapy. 2003, 47, 2636-2639; Surolia, A. et al; FAS t inhibition of malaria. Biochem. J. 2004, 383, 41-412; Chhibber, M. et al; Novel diphenyl ethers: Design, docking studies, synthesis, and inhibition of enoyl ACP reductase of Plasmodium falciparum and Escherichia coli. Bioorg. Med. Chem. 2006, 14, 886-898; Kumar, S. et al; Synthesis and evaluation of substituted pyrazoles: Potential anti-malarials targeting the enoyl ACP reductase of Plasmodium falciparum. Synth. Commun. 2006, 36, 215-226; Sharma, S. K. et al; Green tea catechins potentiate triclosan binding to enoyl-ACP reductase from Plasmodium falciparum (PfENR). J. Med. Chem. 2007, 50, 765-775).

Benzothiophene derivatives are heterocyclic aromatic compounds which have found extensive applications in the pharmaceutical industries. Benzothiophene biphenyl derivatives have been shown to have inhibitory activity against tyrosine phosphatase1B and anti-hyperglycaemic properties (Carl, P. L.; Chakravarty, P. K.; Katzenellenbogen, J. A.; Weber, M. J. Protease activated "prodrugs" for cancer chemotherapy. Proc. Natl. Acad. Sci. U.S.A. 1980, 77, 2224-2228). Raloxifene and arzoxifene are benzothiophene derivatives which act as selective estrogen receptor modulators (SERMs) of clinical value in postmenopausal osteoporosis, treatment of breast cancer and potentially in hormone replacement therapy (Qin, Z.; Kastrati, I.; Ashqodom, R. T.; Lantvit, D. D.; Overk, C. R.; Choi, Y.; van Breemen R. B.; Bolton, J. L.; Thatcher, G. R. Structural modulation of oxidative metabolism in design of improved benzothiophene selective estrogen receptor modulators. Drug Metab. Dispos. 2009, 37, 161-169; Bolognese, M.; Krege, J.H.; Utian, W. H.; Feldman, R.; Broy, S.; Meats, D.L.; Alam, J.; Lakshmanan, M.; Omizo, M. Effects of arzoxifene on bone mineral density and endometrium in post menopausal women with normal or low bone mass. J. Clin. Endocrinol. Metab. 2009, 94, 2284-2289). Benzothiophene class of compounds act as antagonist to retinoid X (RXR) receptors, activator of a TRPV4 (Transient Receptor Potential Vallinoid subtype IV) channel (Thorneloe, K. S.; Sulpizio, A.C.; Lin, Z.; Fiqueroa, D.J.; Clouse, A. K.; McCafferty, G. P.; Chandrimada, T. P.; Lashinger, E.S.; Gordon, E.; Evans, L.; Misajet, B.A.; Demarini, D.J.; Nation, J. H.; Casillas, L.N.; Marquis, R. W.; Vota, B.J.; Sheardown, S.A.; Xu, X.; Brooks, D.P.; Lapping, N. J.; Westfall, T.D. N-((1S)-1-{[4-((2S)-2-[(2,4-Dichlorophenyl)sulfonyl]amino}-3-hydroxypropanoyl)-1-piperazinyl]carbonyl}-3-methylbutyl)-1-benzothiophene-2-carboxamide (GSK116790A), a Novel and Potent Transient Receptor Potential Vanilloid 4 Channel Agonist Induces Urinary Bladder Contraction and Hyperactivity: Part I. J. Pharmacol. Exp. Ther. 2008, 326, 432-442) and also have anti tumor properties (Villar, R.; Encio, I.; Miqliaccio, M. Gil, M.J.; Martinez-Merino, V. Synthesis and cytotoxic activity of lipophillic sulphonamide derivatives of the benzo[b]thiophene 1,1 dioxide. Bioorg. Med. Chem. 2004, 12, 963-968).

Various biodegradable linkages like esters, thioesters, amides have long been used in the formation of prodrugs (Law, B. et al; Proteolysis: A biological process adapted in drug delivery, therapy and Imaging. Bioconjugate Chemistry. 2009, 20, 1683-95; Carl, P. L. et al; Protease activated "prodrugs" for cancer chemotherapy. Proc. Natl. Acad. Sci. U.S.A. 1980, 77, 2224-2228; Smal, M. A. et al; Activation and cytotoxicity of 2-alpha-aminoacyl prodrugs of methotrexate. Biochem. Pharmacol. 1995, 49, 567-574). These prodrugs get activated in presence of enzymes like esterases, thioesterases, amidases, respectively to generate active drugs. Since the active drugs are generated within the cellular milieu, the bioavailability of these compounds are significantly higher.

*Plasmodium* has a complex life cycle in the human host. Malarial sporozoites transmitted by anopheles mosquito enter the blood stream after crossing the cell barriers. It invades the hepatocytes and transforms into liver-stage parasites. These exoerythrocytic forms undergo multiple divisions to form thousands of merozoites (Prudencio, M.; Rodriguez, A.; Mota, M.M. The silent path to thousands of merozoites: the Plasmodium liver-stage. Nat. Rev. Microbiol. 2006, 4, 849-856). Currently, primaquine is the only available drug against liver-stage of the parasite (Valecha, N. et al; Comparative antirelapse efficacy of CDR1 compound 80/53 (Bulaquine) vs primaquine in double blind clinical trial. Curr Sci. 2001, 80, 561-563; Walsh, D. S. et al; Randomized trial of 3-dose regimens of tafenoquine (WR238605) versus low-dose primaquine for preventing Plasmodium vivax malaria relapse. Clin Infect Dis. 2004, 39, 1095-1103). Recently, triclosan, a known Type II FAS inhibitor, has also been shown to inhibit late liver-stages (Singh, A.P.; Surolia, N.; Surolia, A. Triclosan inhibit the growth of the late liver-stage of Plasmodium. IUBMB life 2009, 61, 923-928).

US patent application US2005131058 refers to antimalarial agent having antimalarial activity with little side effects, in particular, having remarkable antimalarial activity against drug-resistant malaria parasites, and being capable of increasing solubility not only to organic solvent including olive oil, but also to water, and therefore, being usable not only as oral drugs but also as injectable solutions.

CN101292983 refers to the pharmaceutical use of (1'-(7"-chlorine-quinoline-4"-radix) piperazine-4'-radix)-3-monoprop for inhibiting the growth of malaria parasites and the fission of the schizont thereof, and can be used for the preparation of anti-malarial drugs.

US2005090480 provides the use of zinc complexes of selected amino acids from D or L isomers of proline, lysine, histidine, glycine, arginine and tryptophan or their various hydroxyl, amino, alkyl and carboxyl derivatives and zinc chloride, zinc acetate or other pharmacologically acceptable salts of zinc. The use of the compound comprises administering an effective amount of said compounds for inhibition of growth: of the malarial parasite, Plasmodium falciparum. The compound is lethal to the parasite in RBC cultures but have no effect on the RBCs.
WO2005037290 refers to the use of phosphono derivatives of selected aliphatic acids represented by the structural formulae R-COOH, R being PO3H2 or CRIR2-PO3H2 where R1 / R2 are H, OH, COOH or alkyl groups for the treatment of malaria.
US2006183167 describes a novel assay method of identifying candidate compounds as anti-malarials based on the property of binding to plasmodial parasite 90 kDa heat shock protein.
US3764604 refers to a series of 4-pyridylcarbinolamines for the treatment of plasmodial infections. The compounds have substituted phenyl groups at positions 2- and 6- on the pyridine moiety, with the electronegative substituents (the same, or different) present on the phenyl nuclei.
CH533123 refers to the use of Quinine and quinidine analogues as anti-malarials, antiarrhythmics and flavourings for drinks.
GB1217427 refers to some pharmaceutical compositions with suitable excipients for oral or parenteral application as anti-malarials and anti-arrhythmic agents.
US2011021467 relates to methods of treating or preventing malaria which comprises administering to a patient in need thereof, an effective amount of 1H-indazole-3-carboxamide derivatives.
CA2726158 provides a class of compounds, pharmaceutical compositions comprising such compounds and methods of using such compounds to treat or prevent malaria.
There remains a need in the art for effective and minimally toxic anti-malarials. There remains a need in the art for chemical compounds capable of inhibiting or killing *Plasmodium.* Further there remains a need in the art for pharmaceutical compositions for use in the treatment of malaria. Since, PfENR is known to be highly expressed and indispensable for liver-stage of the parasite, benzothiophene carboxamide compounds, mentioned in the present invention, were tested on the liver-stages of malaria. Unlike most other drugs which are specific for either erythrocytic or liver-stage, these benzothiophene derivatives have potent anti-malarial activity with effective targets in both red blood cell stage as well as liver-stage. Hence, these compounds hold promise for the development of potent anti-malarials.

## Summary

The present invention relates to benzothiophene carboxamide compounds of formula I, their polymorphs, stereoisomers, prodrugs, solvates, pharmaceutically acceptable salts and formulations thereof, which are useful as COX-2 inhibitor and a PfENR inhibitors,

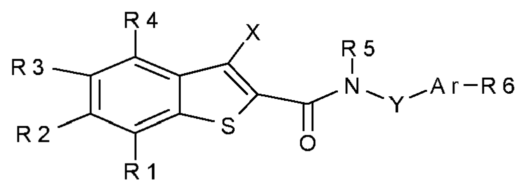

Formula I

wherein

X is a halogen;
Y is $C_1$-$C_6$ alkylene;
Ar is phenyl or naphthyl;
$R_6$ is selected from the group consisting of H, $C_1$-$C_4$ alkyl, $C_1$-$C_4$ alkoxy, OH, halogen, haloalkyl, perfluroalkyl, nitro, cyano and amino; and
$R_1$, $R_2$, $R_3$, $R_4$ and $R_5$ are independently selected from a group consisting of H, $C_1$-$C_4$ alkyl, allyl and $C_2$-$C_6$ alkenyl.

The invention further relates to pharmaceutical compositions containing such compounds and their use as therapeutics agents as anti-malarials anti-inflammatory agent.
These and other features, aspects, and advantages of the present subject matter will become better understood with reference to the following description and appended claims. This summary is provided to introduce a selection of concepts in a simplified form. This summary is not intended to identify key features or essential features of the claimed

subject matter, nor is it intended to be used to limit the scope of the claimed subject matter.

**Brief Description of Figures.**

**Figure 1:** Analgesic activity of known NSAID.
**Figure 2:** Comparison of analgesic activity of compound on hot plate model.
**Figure 3:** Comparison of analgesic activity of compound in Tail Flick Assay.
**Figure 4:** Mechanical allodynia.
**Figure 5:** Anti-Inflammatory activity.
**Figure 6:** Western blot analysis of COX-2 level in rat paw tissue homogenate after treatment with Compounds 4, 6, 8 & 9.
**Figure 7:** Predicted docked energy (MM/GBVI Binding free energy) of the best scoring member of the large cluster obtained for each ligand.
**Figure 8:** Overlay of Indomethacin, Ibuprofen, Compound 4, 6 and 8 with Arachidoic Acid in active site of COX-2
**Figure 9:** Barcode PLIF and Population PLIF of the protein ligand interaction fingerprints.
**Figure 10:** The 3D binding mode and 2D ligand interaction map of two known inhibitor Indomethacin and Ibuprofen and two best compounds 4 and 8.
**Figure 11 a.** Synchronised culture of *Plasmodium falciparum* was 3D7 treated without inhibitor, depicting normal schizont. **b.** and **c.** Synchronised culture of *Plasmodium falciparum* was 3D7 treated with 2.5 μM of compound **6** and compound **7** at the ring stage. **d.** and **e.** Synchronised culture of *Plasmodium falciparum* 3D7 treated with 2.5 μM of compound **10** and compound **11.** Growth of the parasite got arrested at the trophozoite stage
**Figure 12a.** Dose Kinetics of compound **6** for *in vivo* mice model of experimental cerebral malaria. Mice were treated with doses of 0.25 mg/Kg, 0.5 mg/Kg, 1 mg/Kg, 2.5 mg/Kg, 5 mg/Kg and 10 mg/Kg body weight for six days intravenously. Each group had five animals. Average number of days of survival (Y-axis) was plotted against the respective dose **b.** Percentage parasitemia (Y-axis) was plotted against mean days of survival (X-axis) in mice model of experimental cerebral malaria treated with compounds **6,** 7,chloroquine and placebo. c. Percentage parasitemia (Y-axis) was plotted against mean days of survival (X-axis) in mice model of experimental cerebral malaria treated with compounds **10, 11**,chloroquine and placebo.
Each point denotes the mean survival days of five mice. 5 mg/kg body weight of compound was administered daily for six days via intra venous route. Chloroquine was used as the control drug.
**Figure 13.** Compounds **(a) 6** and **(b) 7** docked with PfENR-NAD+ Complex. The inhibitors are shown in Balls and Sticks. NAD and active-site residues are shown in sticks.
Figures were generated using MOE v208.10.
**Figure 14a.** Inhibition of PfENR at various concentrations (0-1 μM) of compound **6.** The percentage activity was calculated from residual activity and was plotted against log concentration.
**Figure 14b.** Inhibition of PfENR at various concentrations (0-1 μM) of compound **7**. The percentage activity was calculated from the residual enzyme activity and was plotted against log concentration.
**Figure 14c.** Inhibition kinetics of compound **6** with respect to NADH .The effect of NADH on inhibition by compound **6** was determined using Dixon plot. 30 nM PfENR was assayed in presence of 200 μM crotonoyl CoA and at two concentrations of NADH, 100 μM [A], 150 μM [●] with various concentrations of compound **6.** Ki of compound **6** was calculated from the X-intercept using equation for competitive kinetics. Each data point indicates three different data sets and the error bar indicates the standard deviation of the data.
**Figure 14d.** Inhibition of PfENR by compound **6** with respect to crotonoyl CoA. PfENR was assayed at two fixed concentrations 200 μM [●] and 300 μM [▲] of crotonoyl CoA in presence of 100 μM of NADH and various concentrations of compound **6.** Ki was calculated from the X-intercept of Dixon plot assuming uncompetitive kinetics. Each data point represents the three independent sets of experiments.
**Figure 15.** Potency assay of compound **6** for PfENR . The potency assay of compound **6** shows the slow onset of inhibition of PfENR by **6.** Curve "a" is control reaction without compound **6** but contained 1% DMSO, curve "b" is inhibition reaction in presence of 115 nM of compound **6,** curve "c" shows further potentiation of inhibition when compound **6** was pre incubated with 35 nM of PfENR for 20 minutes, curve "d" is the inhibition reaction by compound **6** when 10 μM of NAD+ was added to the pre incubated mixture. The Y-axis represents the accumulation of product NAD+ in mM concentration with respect to time (in sec X-axis).
**Figure 16a.** Progress curves of compound **6.** For each concentration of compound **6** progress curve was generated using equation 2a. In the control, standard reaction was set (as described under methods), and contained 1% DMSO apart from other constituents. Compound **6** was added in the reaction mixtures at various concentrations (0-750 nM) as indicated in the figure. The Y-axis represents the accumulation of product NAD+ in mM concentration with

respect to time (in sec X-axis).

**Figure 16b.** Progress curves of compound **7.** For each concentration of compound **7** progress curve was generated using equation 2a. In the control, standard reaction was set (as described under methods), contained 1% DMSO apart from other constituents. Compound **7** was added in the reaction mixtures at various concentrations (0-750nM) as indicated in the figure. The Y-axis represents the accumulation of product NAD+ in mM concentration with respect to time (in sec X-axis).

**Figure 16c.** Dependence of PfENR inhibition on different concentrations of compound **6.** The initial inhibition rate constant (kobs) (Y-axis) for each compound **6** concentration added was calculated from the progress curves using 2a.The kobs thus calculated was fitted to equation 3 with respect to respective compound **6** concentrations and the best fit gave hyperbolic curve demonstrate two phase inhibition mechanism. The error bars represents the standard deviation of the data.

**Figure 16d.** Dependence of PfENR inhibition on different concentrations of compound 7.The initial inhibition rate constant (kobs) (Y-axis) for each compound **7** concentration added was calculated from the progress curves using 2a. The kobs thus calculated was fitted to equation 3 with respect to respective compound 7 concentrations and the best fit gave hyperbolic curve demonstrate two phase inhibition mechanism. The error bars represents the standard deviation of the data.

## Detailed description of the Invention

### Definitions

The following terms as used herein and throughout the present disclosure, have the indicated meaning, unless specifically stated otherwise.

"Alkyl" refers to straight or branched chain having 1 to 10 carbon atoms which is/are further substituted with one or more common substituents including, but are not limited to methyl, ethyl, propyl, isopropyl, butyl, t-butyl and the like.

"Halogen", refers to chloro (Cl), fluoro (F), bromo (Br) and iodo (I).

"Alkenyl" refers to a straight, branched, unsaturated hydrocarbon preferably containing 2 to 10 carbon atoms, and having 1 to 5 double bonds and preferably 1 double bond including, but are not limited to are ethenyl, propenyl, isopropenyl, butenyl, bicycle[2.2.1]heptene and the like.

"Aryl" refers to phenyl or naphthyl.

"Alkoxy" refers to -O-alkyl, wherein alkyl has the meaning as defined herein.

"Prodrug" refers to a derivative of a drug molecule as, for example, esters, carbonates, carbamates, ureas, amides or phosphates that requires a transformation within the body to release the active drug. Prodrugs are frequently, although not necessarily, pharmacologically inactive until converted to the parent drug.

The present invention provides a benzothiophene carboxamide compound of formula I, its polymorph, stereoisomer, prodrug, solvate or pharmaceutically acceptable salt and formulation thereof, wherein said compound is a COX-2 inhibitor or a PfENR inhibitors,

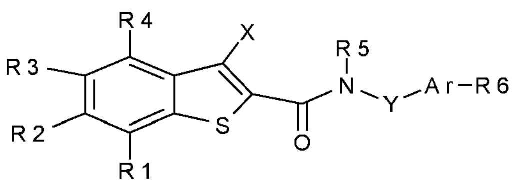

Formula I

wherein

X is a halogen;
Y is $C_1$-$C_6$ alkylene;
Ar is phenyl or naphthyl;
$R_6$ is selected from the group consisting of H, $C_1$-$C_4$ alkyl, $C_1$-$C_4$ alkoxy, OH, halogen, haloalkyl, perfluroalkyl, nitro, cyano and amino; and
$R_1$, $R_2$, $R_3$, $R_4$ and $R_5$ are independently selected from a group consisting of H, $C_1$-$C_4$ alkyl, allyl and $C_2$-$C_6$ alkenyl.

In an embodiment of the present invention, it provodes compounds of formula I wherein $R_1$, $R_2$, $R_3$ and $R_4$ are hydrogen.

In another embodiment it provides compounds of formula I wherein said halogen is bromine.

In yet another embodiment, the compounds of formula I is wherein $R_1$, $R_2$, $R_3$, and $R_4$, are each H; X is Br; $R_5$ is $C_2$-$C_6$ alkenyl; Y is $CH_2$; and $R_6$ is H, halogen or perfluoroalkyl.

Specific embodiments of the present invention are:

*N*-benzyl-3-bromobenzo[b]thiophene-2-carboxamide;
*N*-(4-methoxybezyl)-3-bromobenzo[b]thiophene-2-carboxamide;
*N*-(4-fluorobenzyl)-3-bromobenzo[b]thiophene-2-carboxamide;
*N*-(4-trifluorobenzyl)-3-bromobenzo[b]thiophene-2-carboxamide;
3-bromo-*N*-(2-phenylethyl)-benzo[b]thiophene-2-carboxamide; or
3-bromo-*N*-(naphthalen-1-ylmethyl)-benzo[b]thlophene-2-carboxamide.

Another preferred embodiment provides the compounds:

3-bromo-N-(4-fluorobenzyl)-N-(prop-2-en-1-yl)-benzo[b]thiophene-2-carboxamide; or
3-bromo-N-(prop-2-en-1-yl)-N-[4-(trifluoromethyl)benzyl]-1-benzo[b]thiophene-2-carboxamide.

Another embodiment of the present invention provides compound of formula I for use in treatment of malaria.

Yet another embodiment provides compound of formula I for use in the treatment of inflammation and pain.

Still another embodiment provides compound of formula I wherein the compound has an IC50 value of no more than about 0.115 ± 0.12 for PfENR inhibition.

Yet another embodiment of the present invention provides a pharmaceutical composition comprising a therapeutically effective amount of a compound of formula I with pharmaceutically acceptable excipients.

In another embodiment of the present invention it provides a composition of compound of formula I wherein said composition is for the treatment of malaria.

In yet another embodiment of the present invention it provides a composition of compound of formula I wherein said composition is for the treatment of anti-inflammation and pain.

Another embodiment further provides a method of treatment of inflammation or pain, said method comprising administering a therapeutically effective amount of compound of formula I its polymorph, stereoisomer, prodrug, solvate or pharmaceutically acceptable salt and formulation thereof.

Still another embodiment provides a method of treatment of malaria, said method comprising administering a therapeutically effective amount of compound of formula I, its polymorph, stereoisomer, prodrug, solvate or pharmaceutically acceptable salt and formulation thereof.

In yet another embodiment, the method of treatment of malaria is by inhibiting PfENR enzyme of malaria parasite using compound of formula I, its polymorph, stereoisomer, prodrug, solvate or pharmaceutically acceptable salt and formulation thereof The said parasite is preferably a member of the Plasmodium genus, more preferably, *Plasmodium falciparum.*

In yet another embodiment, the method of treatment of malaria comprises contacting said parasite with an effective amount of a compound of formula I or its polymorph, stereoisomer, prodrug, solvate or pharmaceutically acceptable salt and formulation thereof.

Another embodiment provides a method of killing Plasmodium falciparum parasites in a host mammal comprising administering to the host mammal in need thereof a therapeutically effective amount of a compound of formula I or its polymorph, stereoisomer, prodrug, solvate or pharmaceutically acceptable salt and formulation thereof

Yet another embodiment provides formula I for use in treatment of liver stage malaria parasite. Earlier triclosan was shown to inhibit the liver stage parasite development before invasion with an IC50 of 6.8 μM (Singh A.P. et al Triclosan inhibit the growth of the liver stage of Plasmodium, IUBMB life,_61, 923-928).

Still another embodiment provides a method of treatment of malaria wherein the malaria is treatable by inhibiting the PfENR enzyme of malaria parasite.

An embodiment of the present invention further provides use of a compound of formula I, or its polymorph, stereoisomer, prodrug, solvate or pharmaceutically acceptable salt and formulation thereof, for treatment of malaria.

Another embodiment of the present invention provides use of a compound of formula I, or its polymorph, stereoisomer, prodrug, solvate or pharmaceutically acceptable salt and formulation thereof, for use in the treatment of inflammation and pain.

In a further embodiment, said composition of compound of formula I or its polymorph, stereoisomer, prodrug, solvate or pharmaceutically acceptable salt and formulation thereof, is administered by a route selected from the group consisting of: parenteral, oral, buccal, periodontal, rectal, nasal, pulmonary, transdermal, intravenous, intramuscular, subcutaneous, intradermal, intraoccular, intracerebral, intralymphatic, pulmonary, intraarcticular, intrathecal and intraperitoneal.

In another embodiment, said composition of compound of formula I, or its polymorph, stereoisomer, prodrug, solvate or pharmaceutically acceptable salt and formulation thereof, is formulated into a liquid dispersion form selected from the group consisting of injectable formulations, solutions, delayed release formulations, controlled release formulations, extended release formulations, pulsatile release formulations and immediate release.

In another embodiment, said composition of compound of formula I is formulated into a solid dosage form selected from the group consisting of tablets, coated tablets, capsules, ampoules, suppositories, lyophilized formulations, delayed release formulations, controlled release formulations, extended release formulations, pulsatile release formulations, immediate release and controlled release formulations.

In yet another embodiment of the present invention, compounds of formula I are potential COX-2 inhibitors.

In another embodiment of the present invention it provides a method of treating mammal suffering from inflammation or pain, said method comprising the step of administering a therapeutically effective amount of a benzothiophene carboxamide compound of formula I, or its polymorph, stereoisomer, prodrug, solvate or pharmaceutically acceptable salt and formulation thereof, to said mammals.

In another embodiment of the present invention, benzothiophene carboxamide compounds of formula I, or its polymorph, stereoisomer, prodrug, solvate or pharmaceutically acceptable salt and formulation thereof, are used as anti-inflammatory, wherein the inflammation and pain is associated with bone pain, joint disease, skin, muscle, joints, bones, and ligaments, cuts and sprains, thorax (heart and lungs), abdomen (liver, kidneys, spleen and bowels), pelvis (bladder, womb, and ovaries), dental pain, headache and spondylitis.

Further, the compounds of the present invention are PfENR inhibitors. Since, PfENR is known to be highly expressed and indispensable for liver-stage of the parasite, benzothiophene carboxamide compounds, mentioned in the present invention, were tested on the liver-stages of malaria. Unlike most other drugs which are specific for either erythrocytic or liver-stage, these benzothiophene derivatives have potent anti-malarial activity with effective targets in both red blood cell stage as well as liver-stage. Hence, these compounds hold promise for the development of potent anti-malarials.

In the present invention, the conjugation of carboxyl substituent of bromo-benzothiophene to the primary amine, linked to benzyl derivatives, to form carboxamide linkage is disclosed. Carboxamide linkage can be degraded by various amidases leading to generation of two independent moieties. Bromo-benzothiphene carboxamide class of compounds when further derivatised with benzyl, phenylethyl and naphthyl groups were shown to effectively inhibit the growth of *Plasmodium.*

**EXAMPLES**

The following examples are intended as an illustration of and not a limitation upon the scope of the invention as defined in the appended claims. The compounds of this invention can be prepared by a variety of other synthetic routes known in the art.

__**Scheme 1 for the preparation of compounds of formula I:**__

Reagents and conditions (i)Anhyd DMF, arylalkyl amine, DCC, HOBt, overnight (stirring under $N_2$ atmosphere at RT) (ii) Anhy THF,NaH/60 °C /30min,CH2=CHCH2Br, 48hr (heat with stirring).

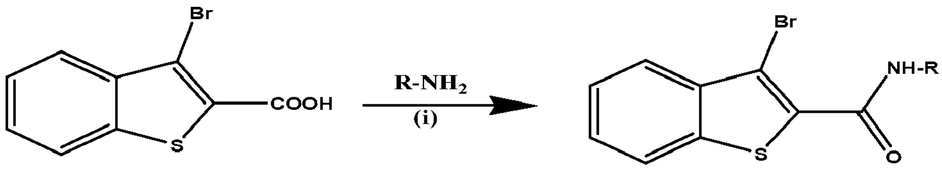


02 04-09

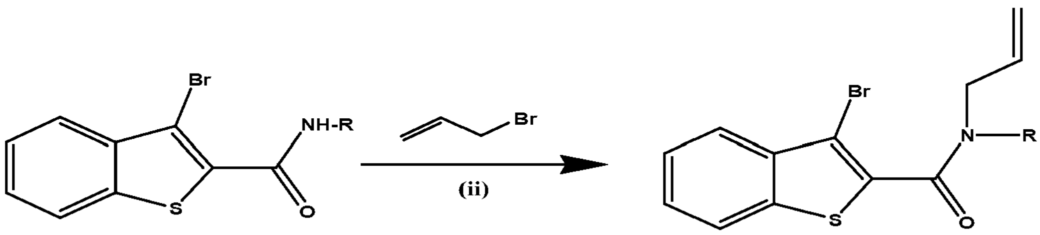


06,07

10,11

| Compound | R |
|---|---|
| 04 | benzyl |
| 05 | 4-methoxybenzyl |
| 06 | 4-fluorobenzyl |
| 07 | 4-trifluorobenzyl |
| 08 | 2-phenylethyl |
| 09 | naphthalene-1-ylmethyl |
| 10 | 4-fuorobenzyl |
| 11 | 4-trifluorobenzyl |

**EXAMPLE 1**

**Synthesis of *N*-benzyl-3-bromobenzo[b]thiophene-2-carboxamide (Compound 4):**

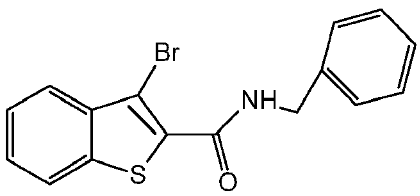

To a cooled mixture (ice bath) of3-bromobenzo[b]thiophene-2-carboxylic acid (02) (0.60 g. 2.33 mmol), benzyl amine (0.24 g, 2.24 mmol), DCC (0.48 g, 2.33 mmol) and HOBt (0.31 g, 2.29 mmol) was added anhydrous DMF (15 mL). The reaction mixture was stirred overnight at room temperature under nitrogen atmosphere till completion of the reaction. The reaction mixture was vacuum filtered to remove precipitated dicyclohexylurea. The filtrate was then evaporated under reduced pressure to give dark oil which was dissolved in ethyl acetate (20 mL) and then re-filtered. The filtrate was vacuum evaporated and the oily residue was purified by column chromatography (30 % ethyl acetate: hexane). To prevent the product from crystallization in the column, a short column was run under pressure to yield *N*-benzyl-3-bromobenzo[b]thiophene-2-carboxamide (0.78 g, 98%) as a colourless, highly crystalline solid. Mp. 115-117 °C. ES-MS m/z: Cald for $C_{16}H_{12}BrNOS$: 346.24 [$M^+$]; Obsd. 346. $^1$H-NMR ($CDCl_3$, 300 MHz): 4.72 (d, 2H, $CH_2$), 7.31-7.45 (m, 5H, ArH benzyl), 7.48 (m, 2H, ArH), 7.83 (dd, 2H, ArH).
$^{13}$C-NMR: 44.5, 106.6, 122.9, 124.7, 125.8, 127.9, 128.0, 129.1, 137.7, 138.6, 138.7 and 161.1.

**EXAMPLE 2**

**Synthesis of *N-(4-methoxybezyl)-3-bromobenzo[b]thiophene-2-carboxamide* (Compound 5)**

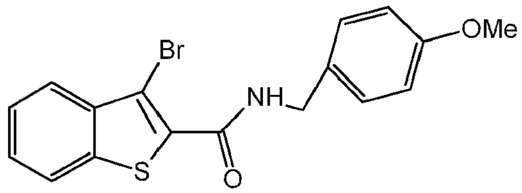

To a stirred solution of 3-bromobenzo[b]thiophene-2-carboxylic acid **(02)** (0.60 g. 2.33 mmol) in anhydrous DMF (15 mL) on ice bath, was added 4-methoxybenzyl amine (0.43 mL, 3.12 mmol), DCC (0.48 g, 2.33 mmol) and HOBt (0.31 g, 2.29 mmol). The reaction mixture was stirred overnight at room temperature under nitrogen and monitored on TLC. Dicyclohexylurea was removed from reaction mixture by vacuum filtration. The filtrate was vacuum evaporated to give dark oil which was dissolved in ethyl acetate (20 mL) and then re-filtered. The filtrate was vacuum evaporated and the oily residue was purified by column chromatography (30 % ethyl acetate: hexane). To prevent the product from crystallization in the column, a short column was run under pressure to yield N-(4-methoxybenzyl)-3-bromobenzo[b] thiophene-2-carboxamide (0.85 g, 3.30 mmol) as a colourless, highly crystalline solid. Mp. 123-124 °C. ES-MS m/z: Cald for $C_{17}H_{14}BrNO_2S$: 376.27 [$M^+$]; Obsd. 376.26. $^1$H-NMR ($CDCl_3$, 300 MHz): 3.80 (s, 3H, OMe), 4.65 (d, 2H, $CH_2$), 6.91 (m, 2H), 7.34 (m, 2H), 7.46 (m, 2H, ArH), 7.83 (d, 2H, ArH).
$^{13}$C-NMR: 44.0, 55.5, 106.6, 114.4, 122.9, 124.7, 125.7, 125.7, 129.4, 135.1, 138.7, 159.4 and 161.0.

**EXAMPLE 3**

**Synthesis of *N-(4-fluorobenzyl)-3-bromobenzo[b]thiophene-2-carboxamide* (Compound 6)**

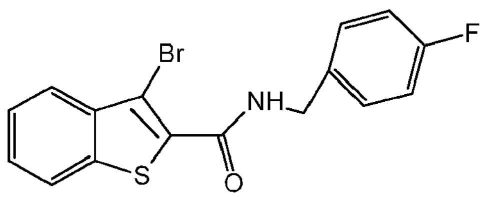

To a cooled mixture (ice bath) of the 3-bromo substituted acid (0.60 g. 2.33 mmol), 4 flurobenzyl amine (0.29 mL, 3.11 mmol) and DCC (0.48 g, 2.33 mmol) and HOBt (0.31 g, 2.29 mmol) was added anhydrous DMF (15 mL). The reaction mixture was stirred overnight at room temperature under nitrogen atmosphere till completion of the reaction. The reaction mixture was vacuum filtered to remove precipitated dicyclohexylurea. The filtrate was then evaporated under reduced pressure to give dark oil which was dissolved in ethyl acetate (20 mL) and then re-filtered. The filtrate was vacuum evaporated and the oily residue was purified by column chromatography (30 % ethyl acetate: hexane). To prevent the product from crystallization in the column, a short column was run under pressure to yield 3-bromo-*N*-(4-fluorobenzyl)-benzo[b]thiophene-2-carboxamide. Mp. 120-125° C. ES-MS m/z: Cald for $C_{16}H_{11}BrNOSF$; 364.23 [$M^+$]; Obsd 364.20. $^1$H-NMR ($CDCl_3$, 300MHz) 4.70 (d, 2H), 7.45 (m, 2H, ArH) 7.83 (dd, 2H, ArH) 7 (m, 2H), 7.36 (m, 2H).
$^{13}$C-NMR 44.0, 106.6, 125.7, 124.7, 123.9, 124.5, 130, 138.7, 163.5 and 161.0.

**EXAMPLE 4**

**Synthesis of *N-(4-trifluorobenzyl)-3-bromobenzo[b]thiophene-2-carboxamide* (Compound 7)**

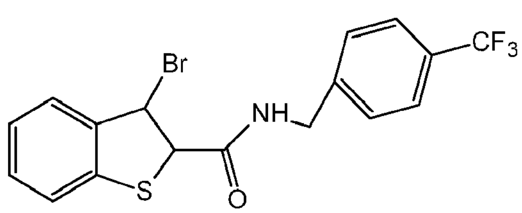

To a cooled solution of 3-bromo substituted acid (.0.60 g. 2.33 mmol), in anhydrous DMF (15 mL) was added DCC (0.48 g, 2.33 mmol) and HOBt (0.31 g, 2.29 mmol). 4-triflurobenzyl amine (0.42 mL, 3.00 mmol) was added to the reaction mixture dropwise over 5minutes. The reaction mixture was brought to room temperature and stirred under nitrogen atmosphere till completion of the reaction. Dicyclohexylurea was vacuum filtered and the filtrate evaporated under reduced pressure to give dark oil which was dissolved in ethyl acetate (20 mL) and then re-filtered. The filtrate was vacuum evaporated and the oily residue was purified by column chromatography (30 % ethyl acetate: hexane). To prevent the product from crystallization in the column, a short column was run under pressure to yield the target compound.Mp. 128-130° C. ES-MS m/z: Cald for $C_{17}H_{11}BrF_3NOS$ 414.24 [$M^+$], Obsd. 414. $^1$H-NMR ($CDCl_3$, 300 MHz): 4.70 (d, 2H), 7.47 (m, 2H, ArH) 7.12 (m, 2H), 7.38 (m, 2H).
$^{13}$C-NMR 44.0, 106.6, 125.7, 124.7, 123.9, 124.5, 130, 138.7 and 161.0.

**EXAMPLE 5**

**Synthesis of *3-bromo-N-(2-phenylethyl)-benzo[b]thiophene-2-carboxamide* (Compound 8)**

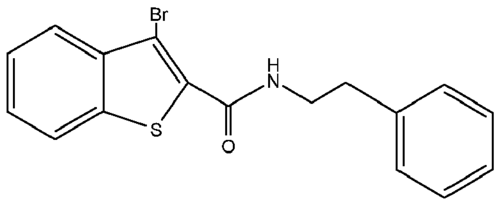

3-bromobenzo[b]thiophene-2-carboxylic acid (02) (0.60 g. 2.33 mmol) was dissolved in anhydrous DMF (15 mL) and cooled on an ice bath. To this stirred solution was added 2-Phenylethyl amine (0.37 mL, 3mmol), DCC (0.48 g, 2.33 mmol) and HOBt (0.31 g, 2.29 mmol). The reaction mixture was stirred overnight at room temperature under nitrogen atmosphere till completion of the reaction. The reaction mixture was vacuum filtered to remove precipitated urea byproduct. The filtrate was vacuum evaporated to give dark oil which was diluted in 20 mL ethyl acetate and then re-filtered. The filtrate was evaporated in vacuo and the oily residue was purified by column chromatography (30 % ethyl acetate: hexane). Since the compound had a tendency to crystallize in column, it was run on short column under pressure to yield the desired product as a colorless solid.ES-MS m/z: Cald for $C_{17}H_{14}BrNOS$ 360.27 [$M^+$]; Obsd 360. $^1$H-NMR ($CDCl_3$, 300 MHz): 3.5 (d, 2H), 3.2 (d, 2H), 7.47 (m, 2H, ArH) 7.12 (m, 2H), 7.38 (m, 2H).
$^{13}$C-NMR 42.9, 35.5, 106.6, 125.7, 124.7, 123.9, 124.5, 130, 138.7 and 161.0.

**EXAMPLE 6**

**Synthesis of *3-bromo-N-(naphthalen-1-ylmethyl)-benzo[b]thiophene-2-carboxamide* (Compound 9)**

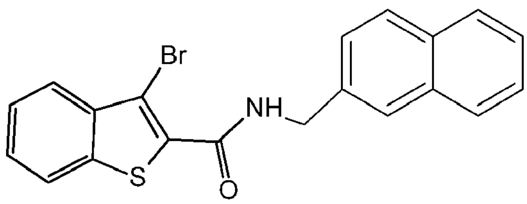

DCC (0.48 g, 2.33 mmol), HOBt (0.31 g, 2.29 mmol) and 1-naphthalene-methylamine (0.431 mL, 3mmol), were added to a cooled solution of 3-bromobenzo[b]thiophene-2-carboxylic acid **(02)** (0.60 g. 2.33 mmol), in anhydrous DMF (15 mL).The reaction mixture was allowed to stir overnight at ambient temperature under nitrogen atmosphere. On completion of the reaction, dicyclohexylurea was removed by filtration. The filtrate was vacuum evaporated to give dark oil. The oily residue was dissolved in 20 ml ethyl acetate and then filtered. The filtrate was evaporated in vacuo. The residue was purified by silica gel chromatography (30 % ethyl acetate: hexane). To prevent the product from crystallization in the column, a short column was run under pressure to yield the target compound as a colorless solid. ES-MS m/z: Cald for $C_{20}H_{14}BrNOS$ 396.30 [$M^+$]; Obsd 396. $^1$H-NMR ($CDCl_3$, 300 MHz): 4.9 (d, 2H), 7.47 (m, 2H, ArH), 7.12 (m,

2H), 7.38 (m, 2H), 7.1 (m, 2H), 7.3 (m, 2H), 7.6 (s, 1H).
$^{13}$C-NMR (75 MHz): 44.0, 125.7, 124.7, 123.9, 124.5, 127.4, 127.5, 128.3, 128.2, 130, 138.7 and 161.0.

**EXAMPLE 7**

**Synthesis of *3-bromo-N-(4-fluorobenzyl)-N-(prop-2-en-1-yl)-benzo[b]thiophene-2-carboxamide* (Compound 10)**

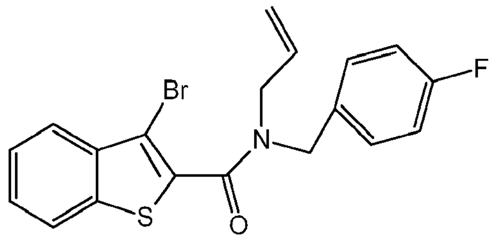

To a solution of *N*-(4-fluorobenzyl)-3-bromobenzo[b]thiophene-2-carboxamide (1mmol) in dry THF(2.5 ml) was added NaH (0.055 g, 2.29 mmol) and heated at 60°C for 30 min. Allyl bromide (1.2 mmol) was added to the resulting mixture and heated at 70 °C for 24 h. NaH(0.055 g, 2.29 mmol) and allyl bromide (0.17g, 1.41 mmol) were further added to the reaction mass and maintained at 70 °C for another 24 h. The solvent was vaccum evaporated, and the residue was extracted with diethyl ether, washed with brine, dried and evaporated under reduced pressure.The residue was purified by column chromatography (elution with 2% EtOAc-hexane) to give a colorless solid.ES-MS m/z: Cald for $C_{19}H_{15}BrFNOS$ 404.30 [$M^+$]; Obsd 403.0. $^1$H-NMR ($CDCl_3$, 300 MHz): 4.70 (d, 2H), 5.18 (d, 2H), 7.45 (m, 2H, ArH), 7.83 (2H, ArH), 7 (m, 2H), 7.36 (m, 2H).
$^{13}$C-NMR (75 MHz): 58.0, 106.6, 125.7, 124.7, 123.9, 124.5, 130, 138.7, 163.5, 136, 137.5 and 161.0.

**EXAMPLE 8**

**Synthesis of *3-bromo-N-(prop-2-en-1-yl)-N-[4-(trifluoromethyl)benzyl]-1-benzo[b]thiophene-2-carboxamide* (Compound 11)**

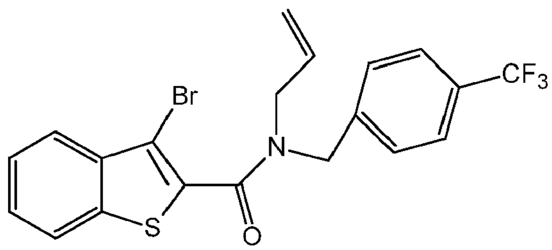

Sodium hydride (0.055 g, 2.29 mmol) was added carefully to a stirred solution of N-(4-trifluorobenzyl)-3-bromobenzo[b]thiophene-2-carboxamide in dry THF(2.5 ml) and heated at 60 °C for 30 min. To the resulting mixture allyl bromide (1.2 mmol) was added and allowed to stir for 24 hrs at 70 °C. Another lot of NaH(0.055 g, 2.29 mmol) and allyl bromide (0.17g, 1.41 mmol) were added to the reaction mass and maintained at 70 °C for 24 hrs. The solvent was vacuum evaporated. The residue was diluted in diethyl ether, washed with brine, dried and evaporated in vacuo. The residue was purified by silica gel chromatography (elution with 2% EtOAc-hexane) to give a colorless solid.ES-MS m/z: Cald for $C_{20}H_{15}BrF_3NOS$ 454.30 [$M^+$]; Obsd 454.4. $^1$H-NMR ($CDCl_3$, 300 MHz): 4.70 (d, 2H), 5.18 (s, 2H), 7.47 (m, 2H, ArH) 7.12 (m, 2H), 7.38 (m, 2H).
$^{13}$C-NMR 58.0, 106.6, 125.7, 124.7, 123.9, 124.5, 130, 136.4, 138.7, 142.2, and 161.0.

**EXAMPLE 9: Biological evaluation of benzothiophene carboxamide derivatives for anti-inflammatory activities:**

The animals were housed in an animal house facility of National Institute of Immunology, India, with controlled temperature (22± 2 °C) under a 12/12 h light/dark cycle. They had free access to food and water *ad libitum.* All experiments

were conducted according to the guidelines of the international association for the study of pain and approved by the institutional ethical committee for animal research. All *in vivo* experiments were carried out on inbred male Wistrat rats (~ 200-320 g body weight). Same age and equal body weight of animals were used according to experiments. No animals were used for more than one experiment. Most popular non-steroidal anti-inflammatory drugs which are widely use for treatment of chronic and inflammatory pain, such as Ibuprofen, Naproxen, Ketoprofen, Aspirin, Nimesulide, Indomethacine were purchased from Cayman Chemicals, USA and used to evaluate the efficacy of drugs in animal model. These drugs and different derivatives of Benzothiophene were dissolved in minimum volume of Dimethyl sulfoxide (DMSO) and further diluted in Phosphate buffer saline prior to use.

**Example10: Assessment of thermal hyperalgesia:**

The anti-nociceptive efficacy of compounds with respect to acute thermal hyperalgesia is determined by using two established methods described an earlier (i) Hot plate latency test and (ii) Tail flick latency test. Hot plate latency test was performed under red light on the metal surface of hot plate analgesia meter (IITC, Life science, CA). Animals were placed individually on the surface of hot plate maintained at a constant temperature 54 $\pm$ 1°C. The anti-nociceptive response was latency recorded from the time when animal was placed on the heated surface until the behavior response show licking of hind paw to avoid thermal nociception. A maximum hot plate latency of 30 sec was used for each animal to prevent paw tissue damage. Base line nociceptive latency was obtained for each animal prior to any drug administration. Thereafter, groups of animals were treated with different concentration of different derivatives of benzothiophene and well known NSAIDs. These drugs were administered intra-peritonially to each animal and control group received normal saline in similar manner after exposure of thermal stimuli to evaluate baseline latency. Subsequent nociceptive response latency for each animal was determined at 30 and 60 min after drug administration. Furthermore, Anti-nociceptive efficacy of selective compounds was monitored at different time intervals up to 8 hrs. In continuation of tail flick latency assay, the anti-nociceptive effect of the compounds was monitored. Rats treated as described above were subjected to determination of thermal nociceptive pain. Rats were confined in restrainer and allowed to accommodate. The tail withdrawal test consisted of immersing the posterior 8-10 cm of the tail in hot water bath maintained at 54 $\pm$ 1 °C. Withdrawal latency for tail flicking was measured. A maximum tail flick latency of 20 sec was permitted to minimize tissue damage. The nociceptive latencies were expressed by maximum possible effect in percent (% MPE) using following formula (Brady & Holtzman, 1984).

$$\%\,MPE = \frac{(\text{Postdrug latency}) - (\text{Predrug latency})}{(\text{Maximum latency}) - (\text{Predrug latency})}\ X\ 100$$

NSAIDs is commonly evaluated by hot plate latency assay whereby animals are exposed to thermal stimulus (54 degree centrigrade) and the time for hind paw licking is measured as behavioural response for pain.

The anti-nociceptive response of various NSAIDs i.e. Ibuprofen, Naproxen, Ketoprofen, Aspirin, Nimuslide, Indomethacin was evaluated by hot plate latency assay in wistar rats and it was observed that ibuprofen proved to be a better anti-nociceptive drug as compared to other NSAIDs 30 minutes post administration **(fig 1).**

The anti-nociceptive response of NSAIDs and synthesized derivatives of Benzothiophene was studied. Anti-nociceptive response is characterized by hot plate latency assay. First, the anti-nociceptive response of some classic/standard NSAIDs (50 mg/kg b. wt.) i.e. Ibuprofen, Naproxen, Ketoprofen, Aspirin, Nimuslide, Indomethacin by hot plate latency assay in 60 minutes post administration was evaluated and it was observed that ibuprofen showed a better anti-nociceptive effect compared to other NSAIDs. Hence, ibuprofen was chosen for comparison. The anti-nociceptive response of ibuprofen at dosage of 25 and 50 mg/kg b. wt. after 30 min and 60 min injection (i.p.) was studied. It was observed that dosage of 50mg/kg b. wt. of ibuprofen score effective anti-nociceptive response in intervals of 30 min and 60 min, indicating that 50 mg/kg b. wt is a suitable dose for the study. The anti-nocicetive response of benzothiophene derivatives (4-11,) individually at different dosages of 1-30 mg/kg b. wt. and found that the response was dose dependent, however no significant difference was observed for dosage > 15mg/kg b.wt.. It was observed that all the animals injected with benzothiophene derivatives 15 mg/kg of b. wt exhibit significant increase MPE index except for compounds-10 and 11 which showed less latency in comparison to other derivatives. The results indicate that benzothiophene derivatives showed optimal anti-nociceptive response at 15mg/kg b. wt. at both time point 30 and 60 min **(Fig. 2).** Interestingly, the comparative MPE index of Ibuprofin (50mg/kg b. wt.) alongwith our compounds (15mg/kg b. wt.) clearly show that compound-6 has preeminent analgesic activity compared to other compounds and this was further corroborated with the time course study. The above results reveal that compounds 4, 6 and 8 have potent analgesic activity at much lower concentration than Ibuprofen.

the anti-nociceptive potential of cmpounds 4-11 was also evaluated by Tail flick latency assay. The animals

treated with different dosage of compounds 4-11 (5, 10 and 15 mg/kg b. wt.) and ibuprofen (50 mg/kg b. wt.) were subjected to tail flick latency assay at 30 min and 60 min of post injection. It was observed that compound 6, 7, 4, 9 and 8 at the dosage of 15 mg/kg b. wt. showed better anti-nociceptive response than the Ibuprofen (50 mg/kg b. wt.) **(Fig. 3).**

The results have shown that benzothiophene derivatives of the present invention are promising candidate molecules as analgesics. Further, their %MPE are much higher than ibuprofen at much lower dose, indicating that the side effects of the benzothiophene derivatives may be less which otherwise is a problem when drug is effective at higher dose. Higher dose means more stress environment on liver or kidneys to detoxify the drug and vice-versa.

**Example 11: Assessment of Mechanical hyperalgesia**

Mechanical hyperalgesia was measured as hind limb withdrawal threshold in response to a mechanical stimulus applied to hind paws of rat using Randall-selitto meter, Randall and Selitto, 1957. To determine the mechanical hyperalgesia, the force was applied with increased pressure at a constant rate until the animal withdrew its limb. The base line nociceptive threshold was measured before induction of hyperalgesia. Moreover, hyperalgesia was induced by carrageenan and then groups of animal were treated with compound 4, compound 6 and compound 8 (15mg/kg body weight) and Ibuprofen (50mg/kg body weight). Control group was treated with physiological saline. The threshold of pain was measured at 1, 2, 3, 4 and 5 h after induction of hyperalgesia. The anti-nociceptive efficacy of the compounds has been evaluated the efficacy with paw withdrawal threshold latency of animals.

Likewise animals were subjected to mechanical allodynia and threshold of analgesia induced by these compounds 4, 6, and 8 (except compound 9 and 10) and ibuprofen was measured. Animals treated with compounds 6, 4 and 8 at dosage of 15 mg/kg b wt. significantly reversed the mechanical hyperalgesia after 1 hrs and reaches the basal level in 5 hrs **(Fig.4).** The result indicates derivatives of Benzothiophene (6, 4 and 8) have better response than the Ibuprofen (50 mg/kg b. wt.).

Thus, results from the above experiments suggest that Benzothiophene derivatives attenuate the anti-nociceptive response effectively at lover dosage than the Ibuprofen.

**Example 12: Assessment of Inflammatory hyperalgesia:**

Anti-inflammatory activities of the compound 4 to compound 11 were evaluated by carrageenan induced inflammatory pain model (Hargreaves et al. 1988). Rats (~250-300 g body weight) were anesthetized by intra muscular injection of Ketamine (100mg/kg) and xylazine (10mg/kg). Inflammation was induced in right hind paw of rats by intraplantar injection of 0.1 ml of 1% (W/V) $\lambda$-carrageenan (Sigma Aldrich Chemical, USA) freshly prepared in sterile saline. Contra lateral hind paws without injection were used as control. Paw volumes of both hind paws of each animal were measured using a plethysmometer (IITC Life Science, USA). To evaluate anti-inflammatory response of derivates of benzothiophene (compounds 4 to 6, and compound 8 and compound 9) rats were treated with doses of 5 mg/kg, 10 mg/kg and 15 mg/kg to each animal after 30 min injection of $\lambda$-carrageenan. The efficacies of compounds were compared with best analgesic drug Ibuprofen (50mg/kg). Control group was received Phosphate buffer saline (pH 7.4) in similar manner. Paw volume of both hind paw were measured at 1, 2, 3, 4, and 5 hrs. The anti-inflammatory response of the test compounds is expressed in percent of paw edema.

To evaluate the anti-inflammatory effect of these compounds the animals received the intraperitoneal injection of these compounds 30 minutes before carrageenan injection in hind paw. 1% carrageenan was found to induce maximal inflammation till 3 hours and the inflammation decreased drastically after 5 hours, so further studies with the compound were done till 5 hour study.

Anti-inflammatory potential of benzothiophene derivatives were evaluated in animal model of inflammatory pain. The intra-plantar injection of 1% carrageenan in right hind paw of animals cause robust inflammation, however; contra lateral left hind paw without injection of carrageenan remained unaffected and served as control. The inflammation crests at 1-5 h and reached basal level at 8 h . First the anti-inflammatory effect of ibuprofen (25 and 50mg/kg b.wt.) was evaluated in 1% carrageenan challenged animals by measuring the paw volume and found that of ibuprofen (50 mg/kg b.wt) significantly diminish inflammatory hyeralgesia by greater than 2 fold at 3 h compared to carrageenan challenged control group. We next studied the anti-inflammatory response of Benzothiophene derivetives compounds 4-11 at doses of 5-15 mg/kg b. wt. and compared with Ibuprofen (50 mg/kg b.wt.). The dose dependent study showed that the lowest dose (5 mg/kg) of Benzothiophene derivatives were unable to inhibit the inflammatory hyperalgesia, however 10 mg/kg and 15 mg/kg of doses significantly reduced the inflammatory hyperalgesia in dose dependent manner **(Fig. 5).** Again the anti-inflammatory effect of these compounds were much better than classic NSAIDs ( Ibuprofen) even at lower dosage. Thus, our results clearly indicate that Benzothiophene compounds are more potent inhibitors of inflammatory hyperalgesia than the classic NSAID ibuprofen. Moreover, histological evaluation of inflamed tissues corroborated with the above results. Infiltration of inflammatory cells including macrophaases, mast cells, neutophills at the site of injury/inflammation decreased drastically after administration of compound 4-11 compared to caragenan control.

**Example 13: Determine the activation of COX1 and COX2 in inflammatory pain:**

Level of COX1 and COX2 were determined in inflamed paw tissue of rats. Hyperalgesia was induced described as earlier. The animals were treated with test compound 4, compound 6, compound 8 and compound 9 (15mg/kg body weight) and sacrificed after 3 hrs. Paw tissue was removed and homogenized in lyses buffer [20 mM Tris (pH-7.5), 150 mM NaCl, 1 mM EDTA, 1mM DTT, 1 mM Sodium vanadate, 1% NP-40, 0.025% SDS, 0.25% Sodium deoxycholate containing protease inhibitors cocktail (Sigma Aldrich, USA). Homogenate was centrifuged at 15,000 xg for 20 min at 4°C. Resulting supernatant was collected and used to determine the level of COX2. The protein concentration in extracts was estimated using BCA protein assay reagent (Sigma Aldrich, USA). The proteins of cell lysates were fractionated on Tris-Glycine buffered 12% SDS PAGE and transferred to PVDF membrane (GE healthcare) by wet electro blotting (Biorad, USA). The membrane was blocked with Tris buffered saline and 0.05% Tween 20 (TBST) containing 5% nonfat milk for 3h at room temperature followed by incubation with primary antibody overnight at 4°C. After washing with TBST, the membrane was incubated with horseradish peroxidase conjugated secondary antibody for 2h at room temperature. Nonspecifically bound secondary antibody was removed from the membrane by several washes with TBST. Immunoreactive reactive protein was detected with chemiluminescence using X-ray film (Amersham Bioscience, USA) and analyzed using geldoc software.

At molecular level the expression of COX-2 was investigated by western blot to confirm whether the anti-inflammatory effect of benzothiophene compounds of the present invention involves COX-2. Immunochemistry of COX-2 clearly showed reduction in the level of COX-2 enzyme and the decrease was almost normalized to control. Carrageenan injected animals on the other hand showed high level of COX-2 (figure 6). COX exists in two isoforms COX-1 and COX-2, out of which COX-2 is inducible form which is a major player in inflammation.

**Example 14: COX-2 inhibition by benzothiophene carboxamide derivatives: Computational Analysis.**

All computational calculations and graphical manipulations were performed on Intel® Pentium®D CPU 2.8 GHz processor, 0.99GB RAM memory with Microsoft Windows XP professional operating system. Ligand molecule preparation, preparation of receptor protein, molecular docking and ligand interaction map generation were performed using software MOE (The Molecular Operating Environment Version 2009.10, software available from Chemical Computing Group Inc.,1010 Sherbrooke Street West, Suite 910, Montreal, Canada H3A 2R7. http://www.chemcomp.com).

Ligand molecules three dimensional structure were prepared using MOE-builder tool, part of MOE suite and were subjected to energy minimization to a gradient of 0.0001 using dielectric constant of 1 and forcefield partial charge were calculated by MMF94x forcefield. All the ligand molecules were saved into a database for molecular docking calculations.

The atomic coordinates of COX-2 used for molecular docking were derived from 2.1 Å X-ray crystal structure of arachidonic acid bound to the cyclooxygenase channel of cyclooxygenase-2 obtained from the RCSB Protein Data Bank (PDB entry:3HS5). The initial coordinates for docking was set in the sequence editor interface part of MOE suite by: (i) removing residues present in chain B [Amino acid sequence, ligands sequence (containing acrylic acid, B-octylglucoside, protoporphyrin IX, ethandiol, alfa-D-mannose and N-acetyl-D-glucosamine residues) and water molecules]. (ii) Water molecules and other ligand residues except arachidonic acid from chain A were also removed. (iii) Receptor protein was protonated by Protonate3D - Macromolecular Protonation State Assignment tool part of MOE suite. (iv) Partial Charge of the receptor was fixed.

The purpose of Molecular docking experiments was to search energetically favorable binding configurations between small flexible ligands and fixed receptor protein. Docking experiments was executed using Molecular docking structure-based design tool a part of MOE suite 2009.10(The Molecular Operating Environment Version 2009.10, software available from Chemical Computing Group Inc.,1010 Sherbrooke Street West, Suite 910, Montreal, Canada H3A 2R7. http://www.chemcomp.com). The molecular docking on COX-2 was carried out by superimposing the energy minimized ligands database on arachidonic acid in the receptor protein-ligand complex. The database were docked by default nonstochastic Triangle Matcher placement method using London dG scoring, followed by forcefield refinement using default configurations (parameters) of docking simulations. Each ligand was docked in the receptor protein and top 100 pose were retained using arachidonic acid as a definition of binding site. The docked poses were exported and visualized in the main MOE window by database browser. The predicted docking score of best scoring member of each ligand was taken for further analysis and ligand interaction map was generated. Molecular Descriptors were calculated for analysis of ligand protein binding mode. Protein ligand interaction fingerprints (PLIF) were generated from output database of docking using PLIF: Protein Ligand Interaction Fingerprints a structure based design tool part of the MOE suite for summarizing the interactions between ligands and proteins using a fingerprint scheme.

The purpose of molecular docking calculations and structural optimization of the complexes obtained was to investigating the possible interaction mode of compounds/inhibitors used in the present study within the COX-2 binding site and to validate the results obtained by in vivo experiments of anti-analgesic as well as anti-inflammatory activity of

compounds. All molecular docking results were stored into output database, the best conformation docked energy (MM/GBVI Binding free energy) of all compounds were taken and comparative graph were generated along with known inhibitors of COX-2 (Indomethacin and Ibuprofen) (Figure 7). Docking results suggested that compound 8, compound 4 and compound 6 binds better than substrate arachidonic acid and known inhibitors and these compounds can be used as promising molecule for anti-analgesic as well as anti-inflammatory activity. The binding modes of these compounds in the active site pocket of COX-2 similar to substrate and known inhibitors. The ligand interaction maps of the compounds were generated and overlay of ligands with arachidonic acid was generated in LigX tool and is shown in Figure 8. The protein ligand interaction fingerprints data revealed that most of the conformations of inhibitors interacted with Tyr 355, Tyr 385, Ser 353, Leu 352, Arg 120, Val 523, Ala 527 and Ser 530, and shown in Figure 9 as barcode PLIF and Population PLIF. The 3D binding mode and 2D ligand interaction map of two known inhibitor Indomethacin and Ibuprofen and two best compounds 4 and 8 shown in Figure 10.

**Example 15 *In vitro* inhibition of *P. falciparum* 3D7 blood-stage parasites**

Benzothiophene carboxamide derivatives of the present invention were checked for their anti-parasitic activity against 3D7 (chloroquine sensitive strain) in an *in vitro* culture. The $IC_{50}$ value for growth inhibition of parasite was found to be 1 ± 0.3 and 1.18 ± 0.2μM for compounds **6** and **7,** respectively (Table Ia,). To find out parasite stage where these compounds act, synchronised ring stage culture of *P. falciparum* was treated with 2.5 μM concentration of compounds **6** and **7.** The growth of the parasite was noted to be arrested at the the trophozoite stage. Nearly 90% of the parasites were observed to be dead (Fig. 11a, 11b and 11c). When compounds **6** and **7** were added at the trophozoite stage the cells could not proceed to the schizont stage indicating the growth inhibition occurs at trophozoite stage.

**Example 16: *In vitro* inhibition of *P. falciparum* 3D7 blood-stage parasites by compounds 10 and 11**

Synchronised culture of *P. falciparum* 3D7 was treated with 2.5 μM compound **10** and **11** at the ring stage. Parasite growth was arrested after 48 hours at the trophozoite stage and could not proceed to the schizont stage. At 2.5 μM, 40% of the parasites treated with compound **10** were observed to be dead whereas after treatment with compound **11** around 70% of the parasites were dead (Fig. 11c and d). The $IC_{50}$ value for growth inhibition of parasite was found to be 2.93 ±1.2 and 1.3 ± 0.1 μM for compounds **10** and **11,** respectively (Table Ia).

**Example 18: Dose determination of compound 6 and 7 for treating experimental cerebral malaria in C57BL/6 mice**

C57BL/6 mice were infected with blood-stage *P. berghei ANKA* as discussed in the experimental section. After 24 hours of infection mice were treated intravenously with various doses of inhibitors. It was observed that all control mice developed hypothermia by day 2, ataxia and convulsions by day 3 and died by day 6, whereas inhibitor treated groups had considerably higher longevity (11-24 days). Though each group of mice developed hypothermia by day 4, convulsions and ataxia development were delayed in case of treated mice. Groups treated with 5 mg/kg body weight of compound 6 showed longest survival till 24th day whereas the group treated with 10 mg/kg body weight of drug survived till day 20 (Fig. 12a). The Peter's test showed reduction in the parasitemia levels after day 6 and reached an undetectable level by Day 18. The inhibitor treatment significantly delayed the onset of hypothermia and prevented convulsions, ataxia, paralysis, etc. The mice treated with 2.5 mg/kg body weight survived for 18days. Even at or below 1 mg/kg body weight of inhibitor dose, the mice survived for 11 days, but paralysis and ataxia development occurred on Day 7. Since 5 mg/kg body weight dose of compound 6 had the longest survival and had normal kidney and liver functions (urea, creatinine and glucose levels) we used this dose for further studies.

**Example 19: Determination of route of administration of compound 6 and 7 in experimental cerebral malaria in C57BL/6 mice**

To determine the most effective route of administration, 5 mg/kg body weight of the compounds were administered through intraperitoneal, peroral, subcutaneous and intravenous routes. The mice were treated for 6 days. Under compound 6 administration, it was observed that mice treated via intravenous route survived for longest period (survival 24 days, Fig. 12b and Table III), while those treated subcutaneously and intraperitoneally were less effective compared to intravenous route (survival 21 days). Although oral route was considerably less effective compared to other two routes, it still increased the longevity by 5 days. Compound 7 also showed comparative activity to compound 6. Chloroquine was used as the control drug. Chloroquine too was found to be more effective via intravenous and subcutaneous routes compared to oral administration.

**Example 20: Determination of route of administration of compound 10 and 11 in experimental cerebral malaria in C57BL/6 mice**

The dose response experiment for compound **6** showed that 5mg/kg body was the optimum dosage. Therefore, compounds **10** and **11** were also administered at 5mg/Kg body weight. The compounds were administered through intraperitoneal, peroral, subcutaneous and intravenous routes. Compound **10** and **11** were administered once daily for 6 days. When treated with compound **11,** intravenous route was observed to be most effective. The mean survival days for this treatment group was 16 (Fig. 12c and Table III). Subcutaneous and intraperitoneal administration could prolong the longevity to 14 and 10 days respectively. Oral administration was the least effective but the treatment group still could outlive the control group. Compound **10** was also most effective when administered intravenously and could prolong the survival till 15th day. Chloroquine was used as the control drug.

**Example 21: Docking of Benzothiophene Derivatives with PfENR**

Docked conformations of bromo-benzothiophene carboxamide derivativesof the present invention to PfENR reveals that these molecules occupy the hydrophobic pocket composed of Tyr 267, Ala 272, Tyr277, Gly 313, Pro 314, Phe 368, and Ile 369. Benzyl group was observed to be surrounded by substrate binding loop residues Ala 319, Ala 320 and Ile 323 and another loop from Ala 217 to Val 222. Carbonyl group mimics the carbonyl oxygen of the thioester substrate. Compound **6** has two hydrogen bonding interactions, twenty-eight hydrophobic interactions, sixteen aromatic-aromatic interactions and twelve hydrophobic-hydrophillic interactions (Fig. 13a, Table 2). There are eighty-nine other interactions predominantly van der Waals. Compound **6** also has extensive van der Waal s interaction with NAD+. Benzyl ring of compound **6** makes H-II stacking interaction with nicotinamide ring of NAD+. Its carbonyl group interacts with N7 group of NAD+. Compound **7** has six hydrogen bonding interactions, twenty-nine hydrophobic interactions, eighteen aromatic-aromatic interactions and five hydrophobic-hydrophillic interactions (Fig. 13b, and Table 3). There are ninety-nine other interactions like van der Waals. Compound **7** also has extensive van der Waal's interaction with NAD+ as well as $\Pi$-$\Pi$ stacking interaction with nicotinamide ring of NAD+.

**Example 22: Kinetic inhibition of PfENR**

PfENR activity was determined using standard assay described earlier. The $IC_{50}$ of the three best compounds were in nanomolar ranges and the remaining compounds were active at micromolar concentrations (Fig. 14a, and 14b, Table Ia). Compound **6** ($IC_{50}$= 0.115 $\pm$ 0.12$\mu$M) was found to be most potent. The $IC_{50}$ of other two potent compounds were also in the nanomolar range (Compounds **7** = 0.463 $\pm$ 0.1$\mu$M, **4** = 0.51 $\pm$ 0.33$\mu$M). Compounds **10** and **11** had $IC_{50}$ of 51.98 $\pm$ 0.43 and 52.48 $\pm$ 0.22$\mu$M respectively against purified PfENR.Ki values for the best inhibitor, compound **6** were determined individually against cofactor NADH and substrate crotonoyl CoA. It gave competitive kinetics with NADH and uncompetitive against crotonoyl CoA (Fig. 14c and 14d). The Ki values are indicated in the Table Ib. Analysis of kinetic data showed that these inhibitors compete with NADH for binding to PfENR. On the other hand, compound **6** prefers to bind with PfENR in presence of crotonoyl-CoA, hence it is an uncompetitive inhibitor of the substrate. **Example 23: Cofactor potency assays**

NAD+ was shown to potentiate the binding of compound **6** to PfENR (Fig. 15). The control reaction showed a linear increase in the accumulation of NAD+(curve a), whereas when the reaction was carried out in the presence of compound **6,** accumulation of product decreased (curve b). The accumulation of product further decreased when compound **6** was preincubated with the enzyme (curve c). On addition of NAD+ to this reaction the rate of product accumulation further decreased (curve d). This also indicated that these are slow tight binding inhibitors of PfENR.

**Example 24: Detailed analysis of the progress curves of bromo-benzothiophene carboxamide derived inhibitors for PfENR**

Progress curves for inhibition by varying concentrations of compounds **6** and **7** were examined in detail (Fig. 16a and 16b). From the progress curve analyses, it was inferred that for each concentration of the inhibitors the initial and the steady state velocity decreased exponentially with time. At higher inhibitor concentration, steady state is reached rapidly with a decrease in steady state velocity (vs). It indicates that initially a loose complex of inhibitor, NAD+, and PfENR is formed which slowly isomerises into a more stable tight complex.

Progress curves were analysed using equation 3a from which a series of Kobs values were obtained for each concentration of inhibitor. The determined Kobs values were plotted against respective concentrations of compound **6** and **7** which resulted in hyperbolic curve (Fig. 16c and 16d). Hyperbolic curve indicated biphasic binding of inhibitors again reflecting slow tight binding behaviour of bromo-benzothiophene carboxamide derived inhibitors of PfENR.

**Table Ia.** Inhibitory potencies of benzothiphene derivatives against purified PfENR and red blood cell stages of *Plasmodium falciparum* 3D7.

| **Compound** | **Structure** | Inhibition of PfENR ($IC_{50}$ μM) | *P.falciparum* ($IC_{50}$ μM) |
|---|---|---|---|
| **Triclosan** | 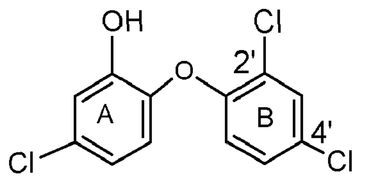 | 0.089 ± .005 | 0.80 ± 0.1 |
| **1** | 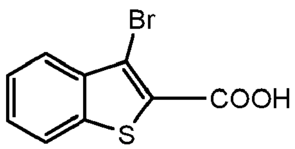 | 1.68 ± 0.045 | 2.1 ± 0.4 |
| **4** | 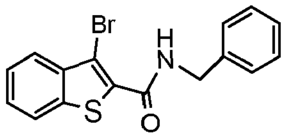 | 0.501±0.033 | 2.00 ± 0.5 |
| **5** | 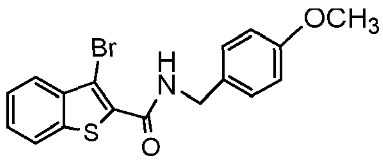 | 31.99 ± 0.23 | 5.93 ± 0.3 |
| **6** | 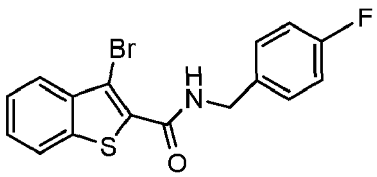 | 0.115 ± .012 | 1.00 ± 0.3 |
| **7** | 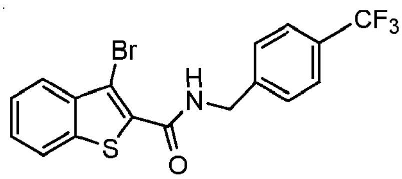 | 0.463 ± 0.01 | 1.18 ± 0.2 |
| **8** | 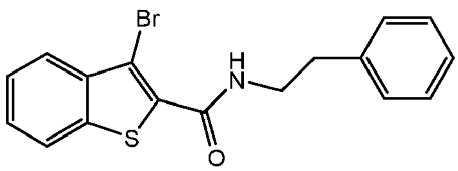 | 54.98 ± 0.42 | 2.46 ± 0.7 |
| **9** | 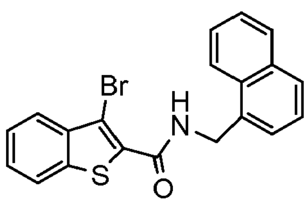 | 52.56 ± 0.33 | 2.45 ± 0.9 |

(continued)

| **Compound** | **Structure** | Inhibition of PfENR ($IC_{50}\mu M$) | *P.falciparum* ($IC_{50}$ $\mu M$) |
|---|---|---|---|
| **10** | 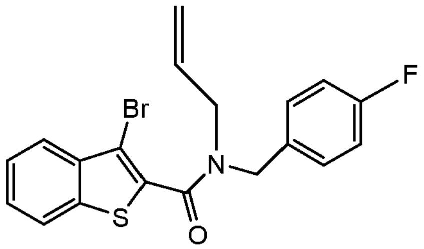 | 51.98 ± 0.43 | 2.93 ± 1.2 |
| **11** | 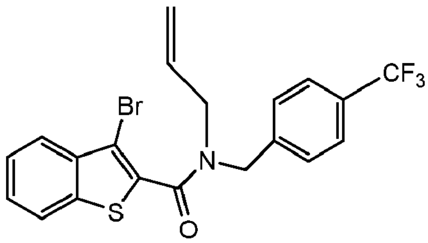 | 52.48 ± 0.22 | 1.3 ± 0.1 |

**Table Ib.** Dissociation constant, isomerization rate constant, dissociation rate constant of compounds **6** and **7** against purified PfENR.

| Compounds | Dissociation constant ($K_i$) nM | Isomerization rate constant ($k_5$ * $10^{-2}$) ($s^{-1}$) | Dissociation rate constant ($k_6$* $10^{-3}$) ($s^{-1}$) |
|---|---|---|---|
| 6 | 48.87 | 1.19±.045 | 1±.0004 |
| 7 | 86.1 | 1.65±.023 | 6.9±.0003 |

**Table II.** Inhibitory potency of compound 6 on hepatic cell stages of *P. berghei.*

| **$IC_{50}$ for EEFs*** | **$TC_{50}$ for HepG2 cells **** | **Therapeutic Index (TI) [=$TC_{50}$/$IC_{50}$]** |
|---|---|---|
| | | |
| 51 μM | 2.0±0.2 mM | 39.0±4 |
| *value for two days treatment **value for one day treatment | | |

**Table III.** Mean survival days depending on different routes of inhibitor/drug administration

| Route of Administration | Compound 6 | Compound 7 | Compound10 | Compound 11 | Chloroquine | Placebo |
|---|---|---|---|---|---|---|
| | Mean Survival Days (Observed for 25 days) | | | | | |
| Intraperitoneal | 20 ± 0.9 | 21 ± 1.3 | 11 ± 0.8 | 10 ± 1.2 | 20 ± 1.5 | 6 ± 0.3 |
| Per oral | 12 ± 1.2 | 11 ± 0.9 | 7± 1.2 | 7± .6 | 17 ± 1.4 | 5 ± 0.2 |
| Subcutaneous | 18 ± 1.3 | 15 ± 1.2 | 12± 1.6 | 14± 1.2 | 21 ± 1.7 | 6 ± 0.3 |
| Intravenous | 24 ± 2.0 | 24 ± 2.1 | 15 ± 0.7 | 16 ± 1.5 | 25 ± 2.1 | 6 ± 0.4 |

Although the subject matter has been described in considerable detail with reference to certain preferred embodiments thereof, other embodiments are possible. As such, the spirit and scope of the appended claims should not be limited to the description of the preferred embodiment contained therein.

## Claims

1. A benzothiophene carboxamide compound of formula I, its polymorph, stereoisomer, prodrug, solvate or pharmaceutically acceptable salt and formulation thereof, wherein said compound is a PfENR inhibitor,

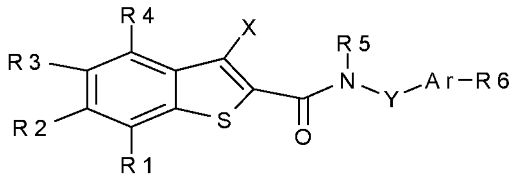

Formula I

wherein

X is a halogen;
Y is $C_1$-$C_6$ alkylene;
Ar is phenyl or naphthyl;
$R_6$ is selected from the group consisting of H, $C_1$-$C_4$ alkyl, $C_1$-$C_4$ alkoxy, OH, halogen, haloalkyl, perfluroalkyl, nitro, cyano and amino; and
$R_1$, $R_2$, $R_3$, $R_4$ and $R_5$ are independently selected from a group consisting of H, $C_1$-$C_4$ alkyl, allyl and $C_2$-$C_6$ alkenyl.

2. The compound as claimed in claim 1 wherein $R_1$, $R_2$, $R_3$ and $R_4$ are hydrogen.

3. The compound as claimed in claim 1 wherein said halogen is bromine.

4. The compound as claimed in claim 1 wherein $R_1$, $R_2$, $R_3$, and $R_4$, are each H; X is Br; $R_5$ is $C_2$-$C_6$ alkenyl; Y is $CH_2$; and $R_6$ is H, halogen or perfluoroalkyl.

5. The compound as claimed in claim 1, which is:

*N*-benzyl-3-bromobenzo[b]thiophene-2-carboxamide;
*N*-(4-methoxybenzyl)-3-bromobenzo[b]thiophene-2-carboxamide;
*N*-(4-fluorobenzyl)-3-bromobenzo[b]thiophene-2-carboxamide;
*N*-(4-trifluorobenzyl)-3-bromobenzo[b]thiophene-2-carboxamide;
3-bromo-*N*-(2-phenylethyl)-benzo[b]thiophene-2-carboxamide; or
3-bromo-*N*-(naphthalen-1-ylmethyl)-benzo[b]thiophene-2-carboxamide.

6. The compound as claimed in claim 1 selected from:

3-bromo-*N*-(4-fluorobenzyl)-*N*-(prop-2-en-1-yl)-benzo[b]thiophene-2-carboxamide; or
3-bromo-*N*-(prop-2-en-1-yl)-*N*-[4-(trifluoromethyl)benzyl]-1-benzo[b]thiophene-2-carboxamide.

7. The compound of formula I as claimed in any of the claims 1 to 6 for use in treatment of malaria.

8. The compound of formula I as claimed in any of the claims 1 to 6, wherein the compound has an IC50 value of no more than about 0.115 $\pm$ 0.12 for PfENR inhibition.

9. A pharmaceutical composition comprising a therapeutically effective amount of a compound of formula I as claimed in any of the claims 1 to 6 with pharmaceutically acceptable excipients.

10. The composition of claim 9 wherein said composition is for the treatment of malaria.

11. A method of treatment of malaria, said method comprising administering a therapeutically effective amount of compound as claimed in any of the claims 1 to 6, its polymorph, stereoisomer, prodrug, solvate or pharmaceutically acceptable salt and formulation thereof

**12.** The method of claim 11 wherein said method is by inhibiting PfENR enzyme of malaria parasite using compound as claimed in any of the claims 1 to 6, its polymorph, stereoisomer, prodrug, solvate or pharmaceutically acceptable salt and formulation thereof

**13.** The method of claim 11, wherein said parasite is a member of the Plasmodium genus.

**14.** The method of claim 11, wherein the parasite is Plasmodium falciparum.

**15.** A method of killing a Plasmodium falciparum parasite, said method comprising contacting said parasite with an effective amount of a compound as claimed in any of the claims 1 to 6 or its polymorph, stereoisomer, prodrug, solvate or pharmaceutically acceptable salt and formulation thereof.

**16.** A method of killing Plasmodium falciparum parasites in a host mammal comprising administering to the host mammal in need thereof a therapeutically effective amount of a compound as claimed in any of the claims 1 to 6 or its polymorph, stereoisomer, prodrug, solvate or pharmaceutically acceptable salt and formulation thereof

**17.** The method of claim 11, wherein the malaria is treatable by inhibiting the PfENR enzyme of malaria parasite.

**18.** Use of a compound as claimed in any of the claims 1 to 6 for treatment of malaria.

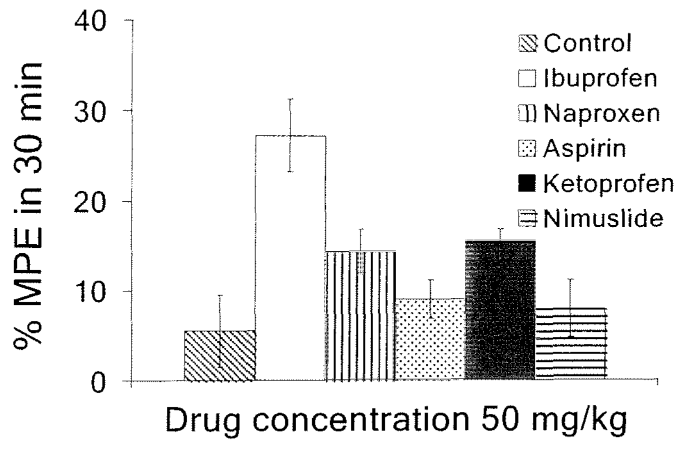


FIGURE 1

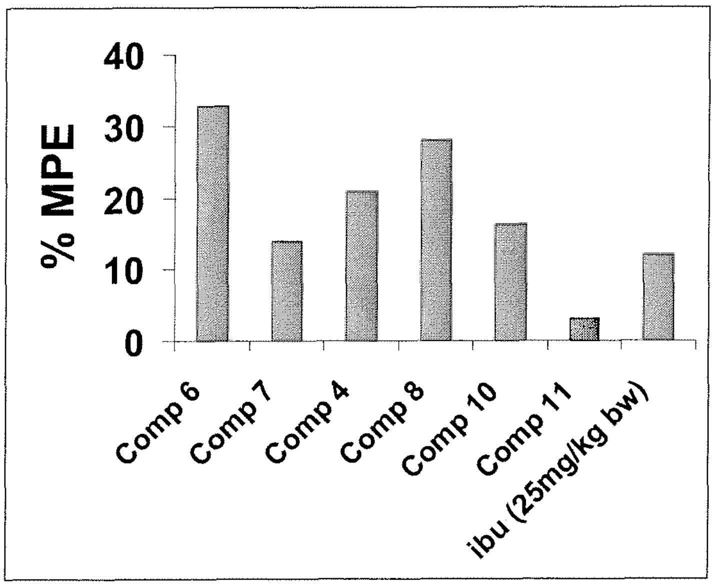


FIGURE 2

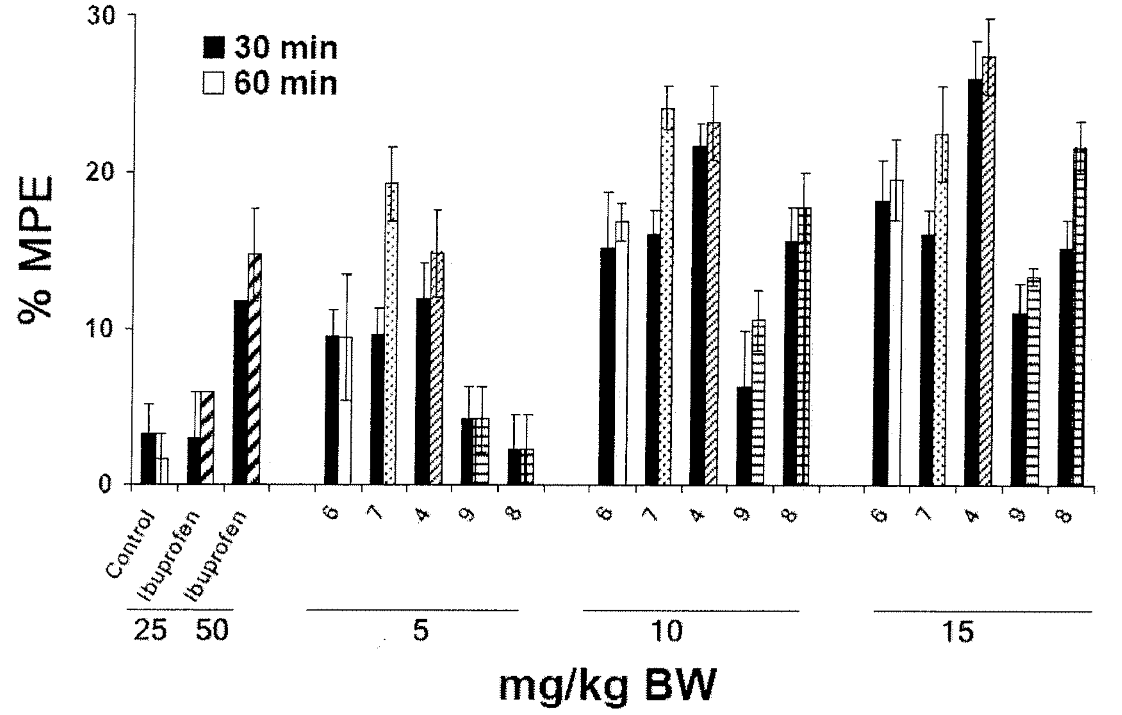


FIGURE 3

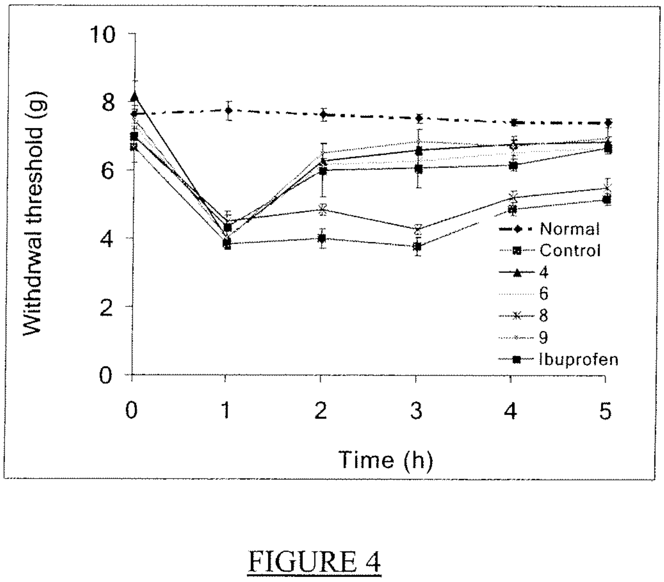


FIGURE 4

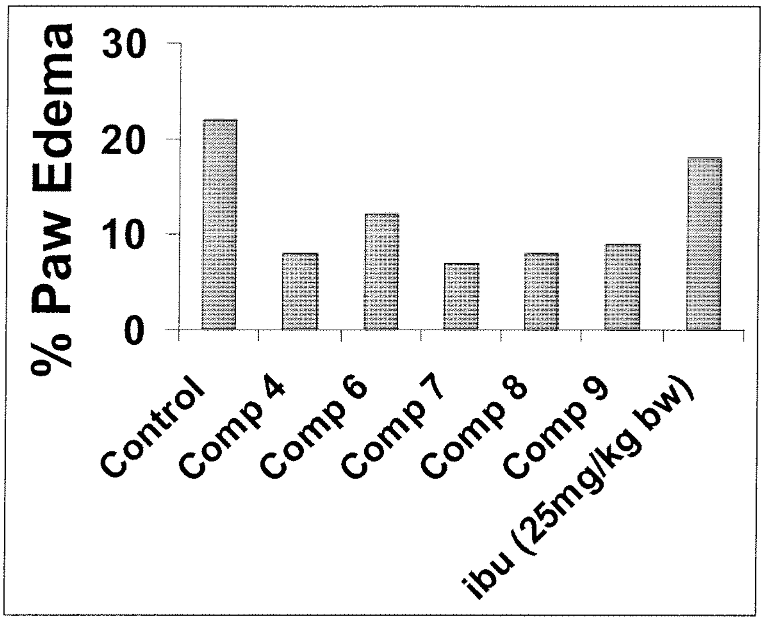


FIGURE 5

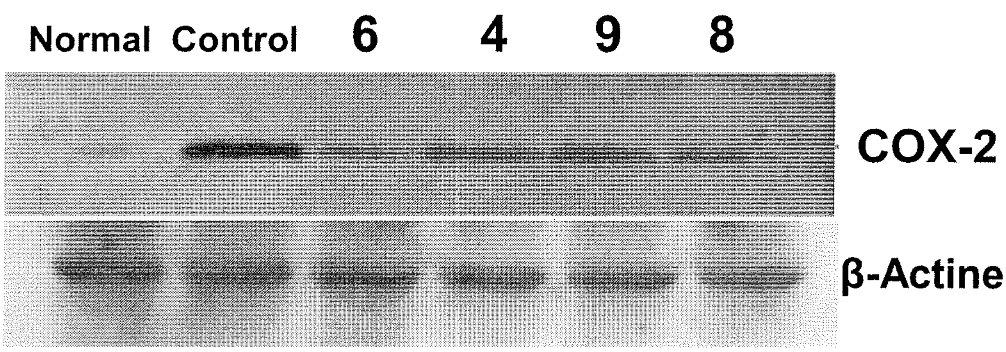


FIGURE 6

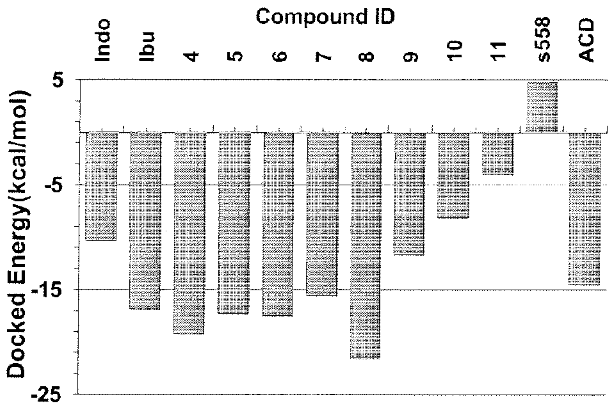


FIGURE 7

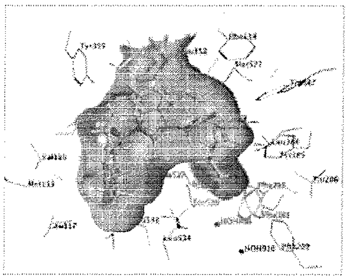

Overlay of Indomethacin with Arachidoic Acid in Active site of Cox-2

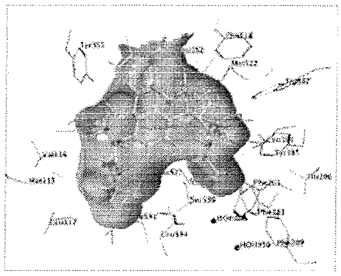

Overlay of Ibuprofen with Arachidoic Acid in Active site of Cox-2

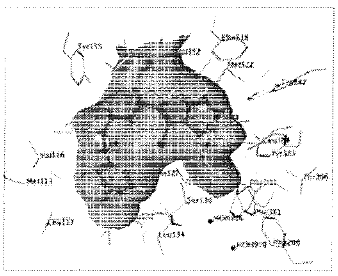

Overlay of Compound 8 with Arachidoic Acid in Active site of Cox-2

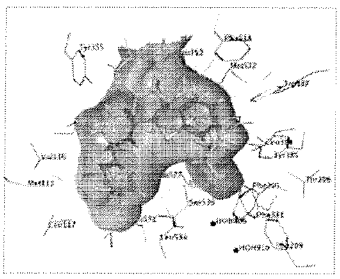

Overlay of Compound 4 with Arachidoic Acid in Active site of Cox-2

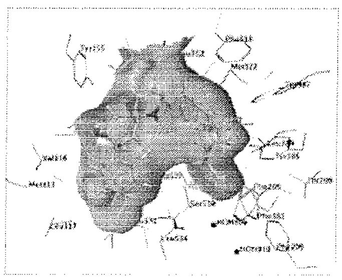

Overlay of Compound 6 with Arachidoic Acid in Active site of Cox-2

FIGURE 8

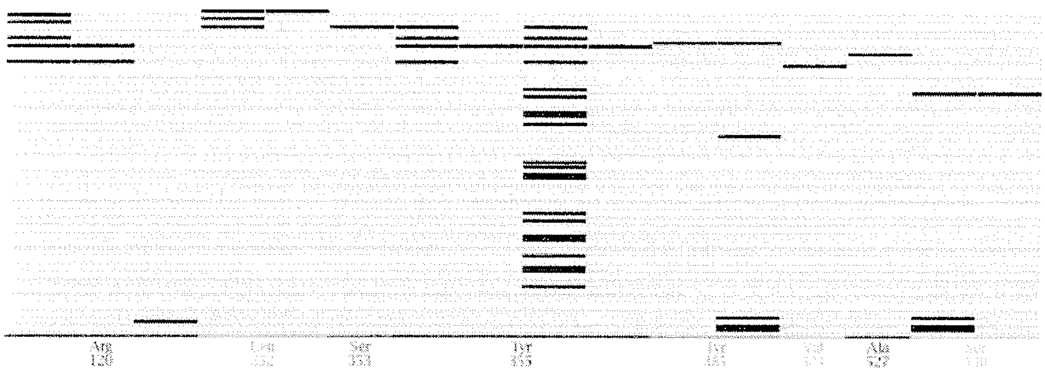


Barcode PLIF

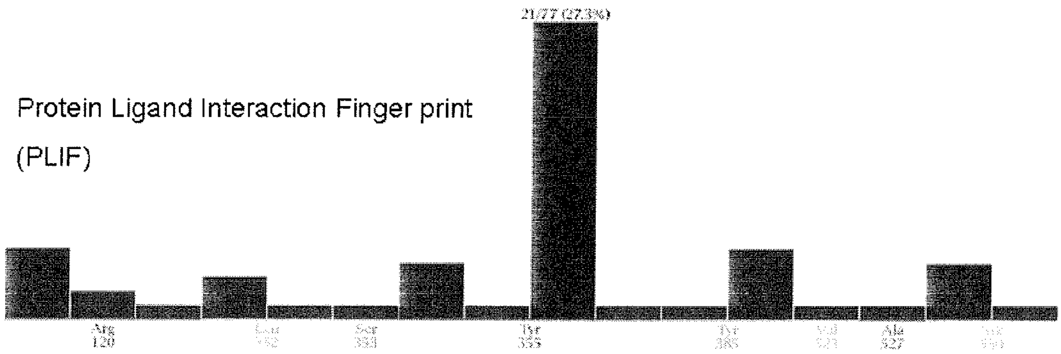


Population PLIF

Figure 9

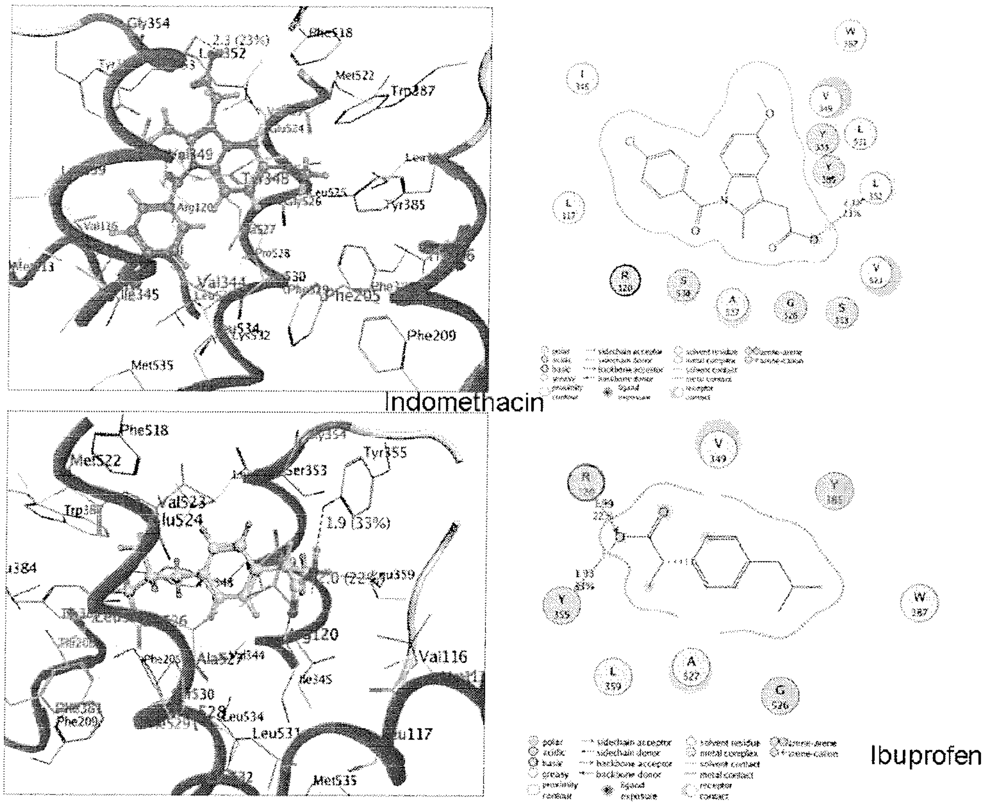


Figure 10

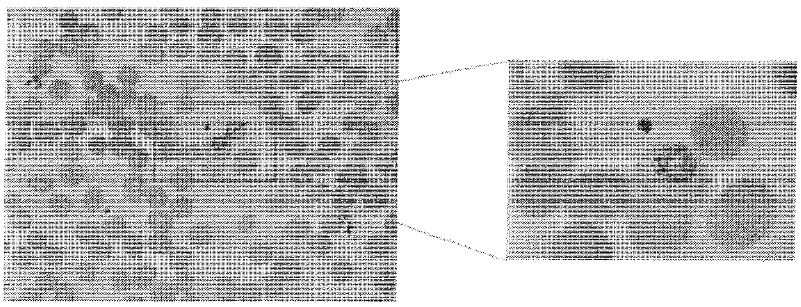

Figure 11a

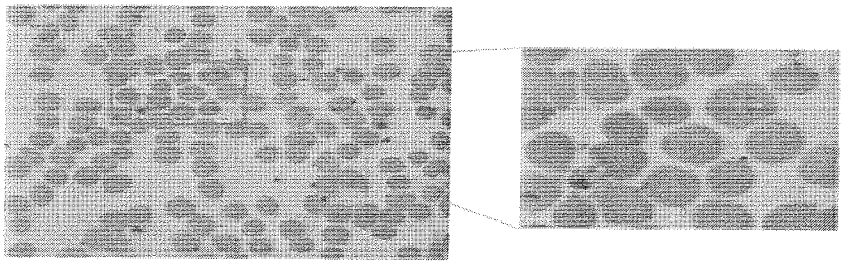

Figure 11 b

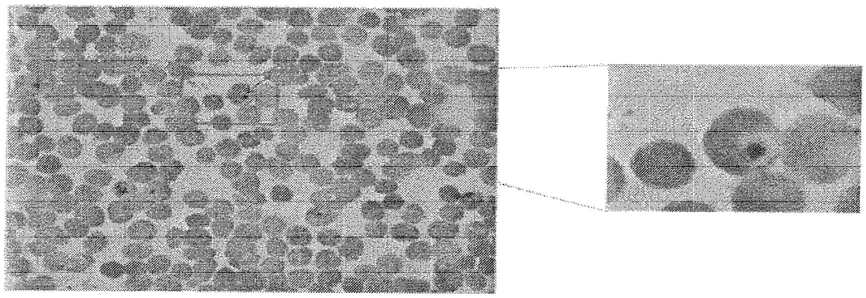

Figure 11 c

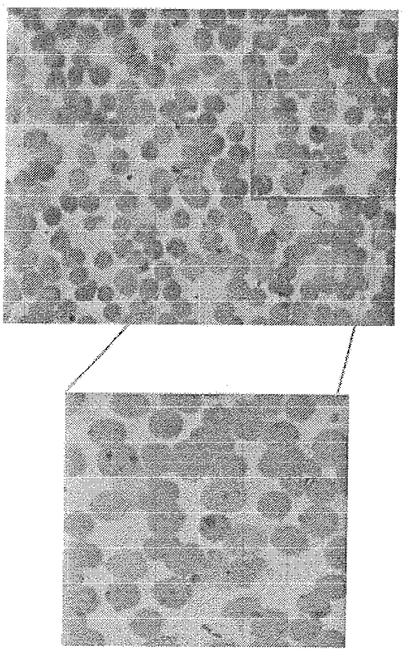

Fig. 11d.

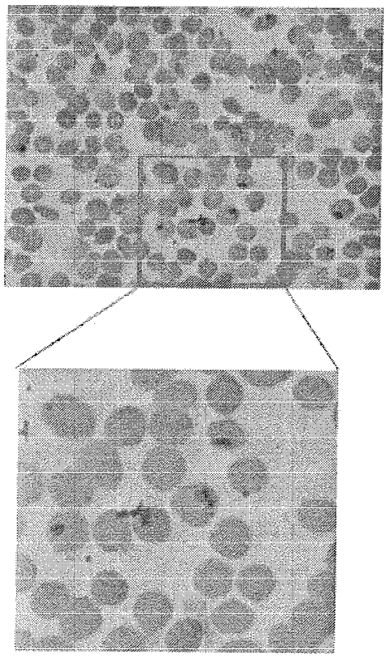

Fig. 11e.

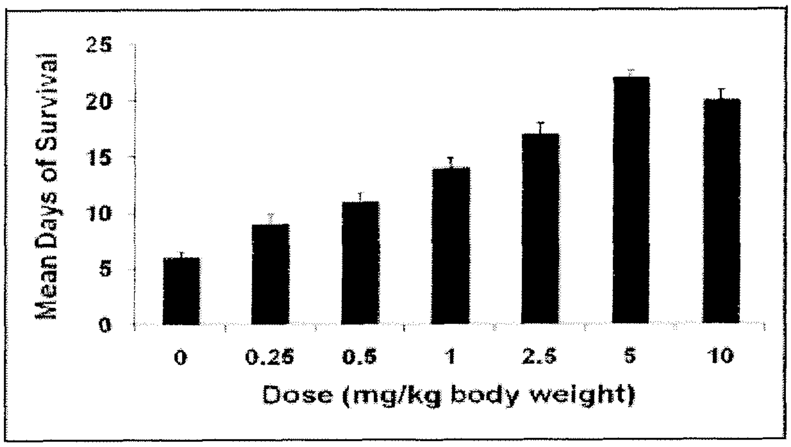


Figure 12 a

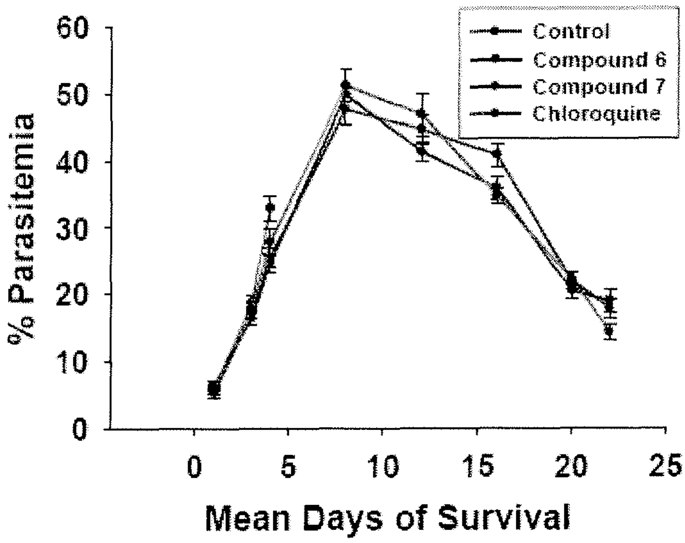


Figure 12 b

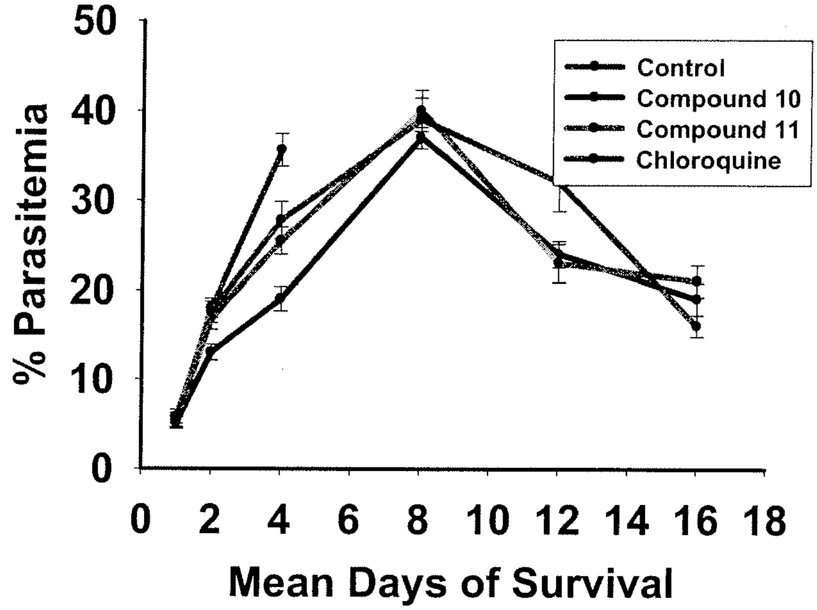


Fig. 12 c

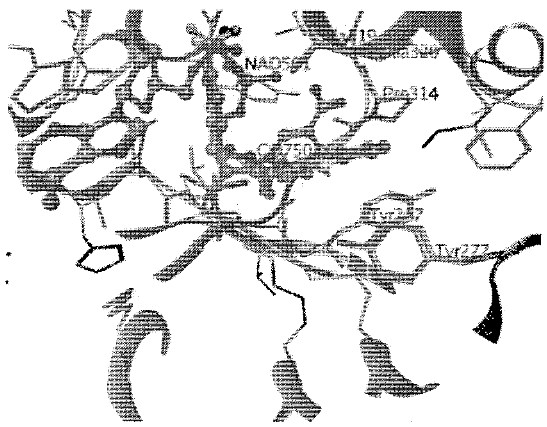


Figure 13 a

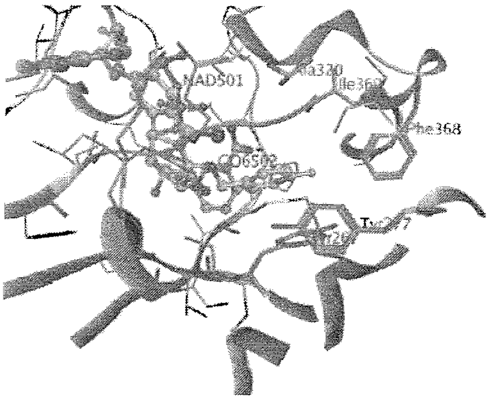

Figure 13 b

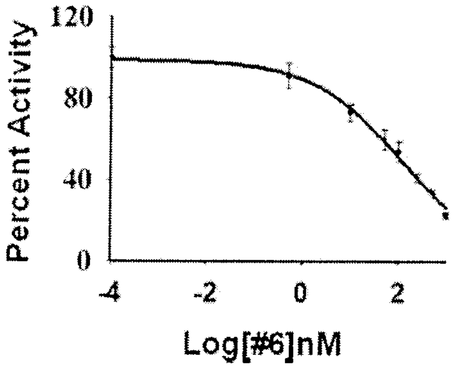


Figure 14a

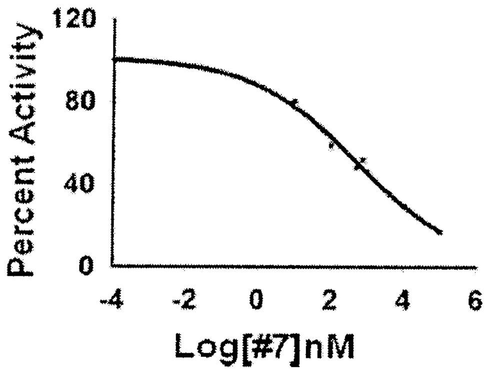


Figure 14 b

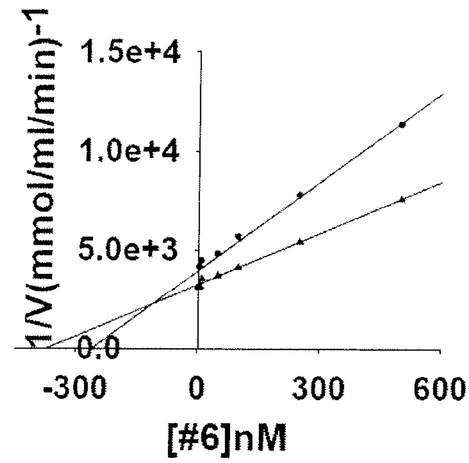


Figure 14 c

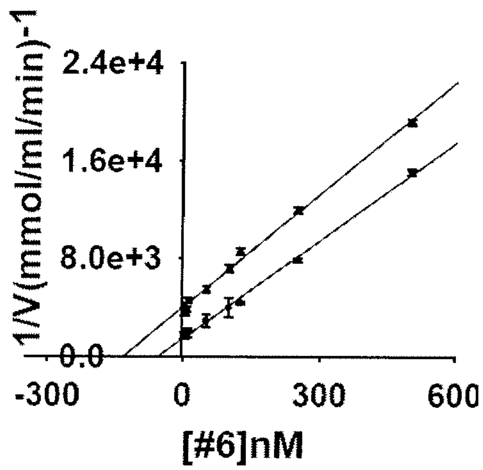


Figure14 d

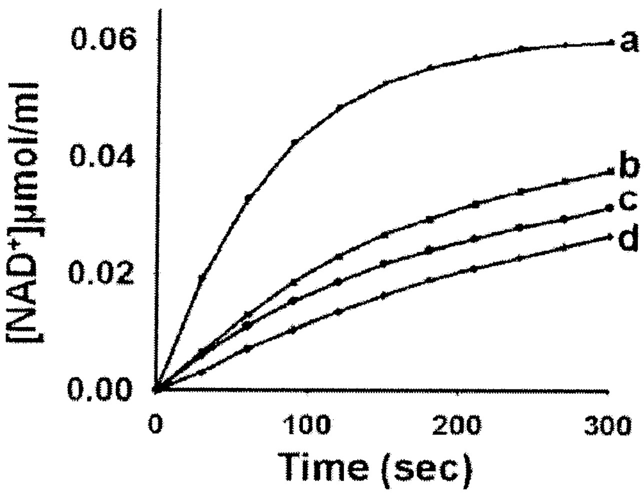


Figure 15

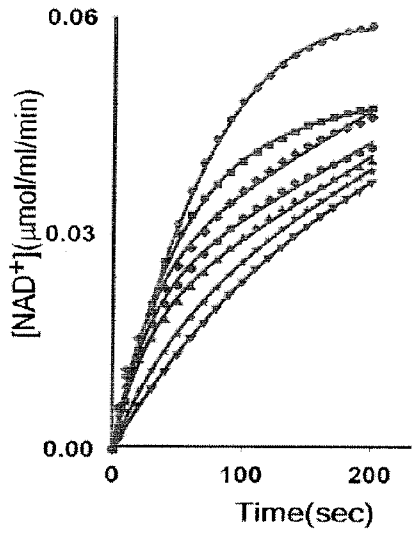


Figure 16a

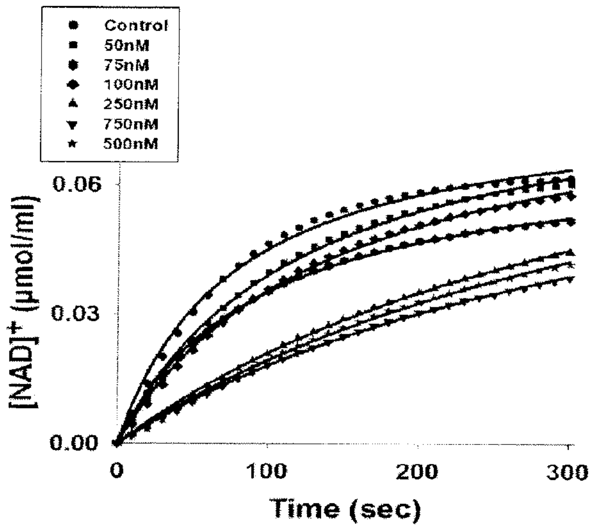


Figure 16b

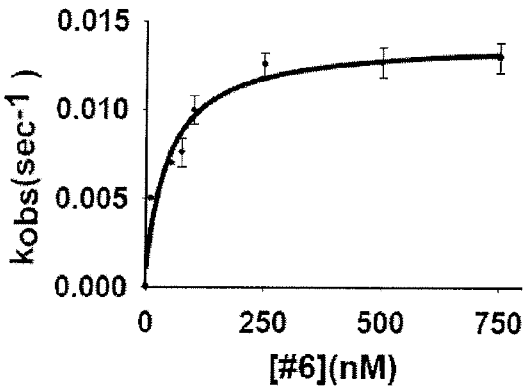


Figure 16c

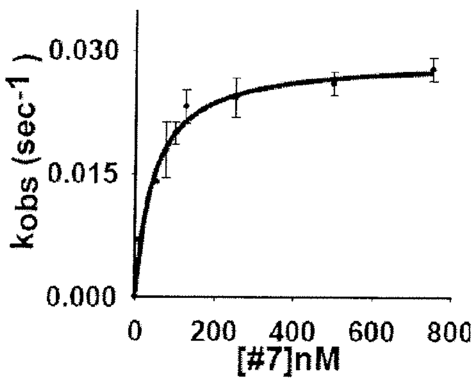


Figure 16d

Europäisches Patentamt
European Patent Office
Office européen des brevets

# EUROPEAN SEARCH REPORT

Application Number
EP 11 17 3782

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/200461 A1 (ANDERSON RICHARD [US] ET AL) 21 August 2008 (2008-08-21) * compounds 275, 349, 355, 387 * | 1-3,8,9 | INV. C07D333/70 A61K31/381 A61P33/06 |
| X | WO 2007/138112 A2 (DEVGEN NV [BE]; BLOM PETRA [BE]; DEFERT OLIVIER [FR]; KALETTA TITUS [B) 6 December 2007 (2007-12-06) * compounds 43, 47, 48, 58 * | 1,8,9 | |
| X | US 2009/280483 A1 (KITAMURA TOSHIO [JP] ET AL) 12 November 2009 (2009-11-12) * formula 12 * | 1 | |
| A | FREUNDLICH J S ET AL: "Synthesis, biological activity, and X-ray crystal structural analysis of diaryl ether inhibitors of malarial enoyl acyl carrier protein reductase. Part 1: 4'-Substituted triclosan derivatives", BIOORGANIC & MEDICINAL CHEMISTRY LETTERS, PERGAMON, ELSEVIER SCIENCE, GB, vol. 15, no. 23, 1 December 2005 (2005-12-01), pages 5247-5252, XP025313827, ISSN: 0960-894X, DOI: 10.1016/J.BMCL.2005.08.044 [retrieved on 2005-12-01] * tables 1-3 * | 1-18 | TECHNICAL FIELDS SEARCHED (IPC) C07D |
| | -/-- | | |

The present search report has been drawn up for all claims

1

| Place of search | Date of completion of the search | Examiner |
|---|---|---|
| Munich | 4 March 2013 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 17 3782

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FREUNDLICH J S ET AL: "Synthesis and biological activity of diaryl ether inhibitors of malarial enoyl acyl carrier protein reductase. Part 2: 2'-Substituted triclosan derivatives", BIOORGANIC & MEDICINAL CHEMISTRY LETTERS, PERGAMON, ELSEVIER SCIENCE, GB, vol. 16, no. 8, 15 April 2006 (2006-04-15), pages 2163-2169, XP025106931, ISSN: 0960-894X, DOI: 10.1016/J.BMCL.2006.01.051 [retrieved on 2006-04-15] * tables 1-5 * ----- | 1-18 | |
| A | KUMAR G ET AL: "Discovery of a Rhodanine Class of Compounds as Inhibitors of Plasmodium falciparum Enoyl-Acyl Carrier Protein Reductase", JOURNAL OF MEDICINAL CHEMISTRY, AMERICAN CHEMICAL SOCIETY, US, vol. 50, 31 May 2007 (2007-05-31), pages 2665-2675, XP002549914, ISSN: 0022-2623, DOI: 10.1021/JM061257W [retrieved on 2007-05-04] * tables 1-4 * ----- | 1-18 | TECHNICAL FIELDS SEARCHED (IPC) |
| X,P | WO 2011/072275 A2 (NONO INC [CA]; SUN XIUJUN [CA]; TYMIANSKI MICHAEL [CA]; GARMAN JONATHA) 16 June 2011 (2011-06-16) * supplier object ID BTB01318 * ----- | 1,2,8,9 | |

The present search report has been drawn up for all claims

1

| Place of search | Date of completion of the search | Examiner |
|---|---|---|
| Munich | 4 March 2013 | Bakboord, Joan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 11 17 3782

This annex lists the patent family members relating to the patent documents cited in the above-mentioned European search report.
The members are as contained in the European Patent Office EDP file on
The European Patent Office is in no way liable for these particulars which are merely given for the purpose of information.

04-03-2013

| Patent document cited in search report | Publication date | Patent family member(s) | Publication date |
|---|---|---|---|
| US 2008200461 A1 | 21-08-2008 | US 2008200461 A1 | 21-08-2008 |
| | | WO 2008103354 A2 | 28-08-2008 |
| WO 2007138112 A2 | 06-12-2007 | NONE | |
| US 2009280483 A1 | 12-11-2009 | JP 4882076 B2 | 22-02-2012 |
| | | US 2009280483 A1 | 12-11-2009 |
| | | WO 2007060715 A1 | 31-05-2007 |
| WO 2011072275 A2 | 16-06-2011 | AU 2010327936 A8 | 28-06-2012 |
| | | CA 2781888 A1 | 16-06-2011 |
| | | EP 2521724 A2 | 14-11-2012 |
| | | US 2011251182 A1 | 13-10-2011 |
| | | WO 2011072275 A2 | 16-06-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7375130 B
- US 7351735 B
- US 7148240 B
- US 6949583 B
- US 6946483 B
- US 4663347 A
- US 4745127 A
- US 4822803 A
- US 4933351 A
- US 4621091 A
- US 4548948 A
- US 6433005 B
- US 2005131058 A
- CN 101292983
- US 2005090480 A
- WO 2005037290 A
- US 2006183167 A
- US 3764604 A
- CH 533123
- GB 1217427 A
- US 2011021467 A
- CA 2726158


### Non-patent literature cited in the description


- **WOLFE, M.M. et al.** Gastrointestinal toxicity of non-steroidal anti-inflammatory drugs. *N Engl J Med.,* 1999, vol. 340, 1888-1899
- **ONG, C.K. et al.** Combining paracetamol (acetaminophen) with nonsteroidal antiinflammatory drugs: a qualitative systematic review of analgesic efficacy for acute postoperative pain. *Anesth Analg.,* 2010, vol. 110, 1170-9
- **SMITH, S. et al.** Structural and functional organization of the animal fatty acid synthase. *Prog. Lipid Res.,* 2003, vol. 42, 289-317
- **RALPH, S. A. et al.** Metabolic maps and functions of the Plasmodium falciparum apicoplast. *Nat. ReV. Microbiol.,* 2004, vol. 2, 23-216
- **ROCK, C. O. et al.** Escherichia coli as a model for the regulation of dissociable (type II) fatty acid biosynthesis. *Biochim. Biophys. Acta,* 1996, vol. 132, 1-16
- **SUROLIA, N. et al.** Triclosan offers protection against blood-stages of malaria by inhibiting enoyl-ACP reductase of Plasmodium falciparum. *Nat. Med.,* 2001, vol. 7, 167-173
- **HEATH, R. J.** *Curr. Opin. InVestig. Drugs,* 2004, vol. 5, 46-53
- **SINGH, A.P. et al.** Triclosan inhibit the growth of the late liver-stage of Plasmodium. *IUBMB life,* 2009, vol. 61, 923-928
- **VAUGHAN, A. M. et al.** Type II Fatty Acid Is Essential for Only Late Liver-stage Development. *Cellular Microbiology,* 2009, vol. 11, 56-520
- **MAHMOUDI, N. et al.** In vitro activities of 25 quinolones and fluoroquinolones against liver and blood-stage Plasmodium spp. *Antimicrobial Agents and Chemotherapy,* 2003, vol. 47, 2636-2639
- **SUROLIA, A. et al.** FAS t inhibition of malaria. *Biochem. J.,* 2004, vol. 383, 41-412
- **CHHIBBER, M. et al.** Novel diphenyl ethers: Design, docking studies, synthesis, and inhibition of enoyl ACP reductase of Plasmodium falciparum and Escherichia coli. *Bioorg. Med. Chem.,* 2006, vol. 14, 886-898
- **KUMAR, S. et al.** Synthesis and evaluation of substituted pyrazoles: Potential anti-malarials targeting the enoyl ACP reductase of Plasmodium falciparum. *Synth. Commun.,* 2006, vol. 36, 215-226
- **SHARMA, S. K. et al.** Green tea catechins potentiate triclosan binding to enoyl-ACP reductase from Plasmodium falciparum (PfENR. *J. Med. Chem.,* 2007, vol. 50, 765-775
- **CARL, P. L. ; CHAKRAVARTY, P. K. ; KATZENELLENBOGEN, J. A. ; WEBER, M. J.** Protease activated ''prodrugs'' for cancer chemotherapy. *Proc. Natl. Acad. Sci. U.S.A.,* 1980, vol. 77, 2224-2228
- **QIN, Z. ; KASTRATI, I. ; ASHQODOM, R. T. ; LANTVIT, D. D. ; OVERK, C. R. ; CHOI, Y. ; BREEMEN R. B. ; BOLTON, J. L. ; THATCHER, G. R.** Structural modulation of oxidative metabolism in design of improved benzothiophene selective estrogen receptor modulators. *Drug Metab. Dispos.,* 2009, vol. 37, 161-169
- **BOLOGNESE, M. ; KREGE, J.H. ; UTIAN, W. H. ; FELDMAN, R. ; BROY, S. ; MEATS, D.L. ; ALAM, J. ; LAKSHMANAN, M. ; OMIZO, M.** Effects of arzoxifene on bone mineral density and endometrium in post menopausal women with normal or low bone mass. *J. Clin. Endocrinol. Metab.,* 2009, vol. 94, 2284-2289

- **THORNELOE, K. S. ; SULPIZIO, A.C. ; LIN, Z. ; FIQUEROA, D.J. ; CLOUSE, A. K. ; MCCAFFERTY, G. P. ; CHANDRIMADA, T. P. ; LASHINGER, E.S. ; GORDON, E. ; EVANS, L.** N-((1S)-1-{[4-((2S)-2-[(2,4-Dichlorophenyl)sulfonyl]amino}-3-hydroxypropanoyl)-1-piperazinyl]carbonyl}-3-methylbutyl)-1-benzothiophene-2-carboxamide (GSK116790A), a Novel and Potent Transient Receptor Potential Vanilloid 4 Channel Agonist Induces Urinary Bladder Contraction and Hyperactivity: Part I. *J. Pharmacol. Exp. Ther.,* 2008, vol. 326, 432-442
- **VILLAR, R. ; ENCIO, I. ; MIQLIACCIO, M. ; GIL, M.J. ; MARTINEZ-MERINO, V.** Synthesis and cytotoxic activity of lipophillic sulphonamide derivatives of the benzo[b]thiophene 1,1 dioxide. *Bioorg. Med. Chem.,* 2004, vol. 12, 963-968
- **LAW, B. et al.** Proteolysis: A biological process adapted in drug delivery, therapy and Imaging. *Bioconjugate Chemistry,* 2009, vol. 20, 1683-95
- **CARL, P. L. et al.** Protease activated ''prodrugs'' for cancer chemotherapy. *Proc. Natl. Acad. Sci. U.S.A.,* 1980, vol. 77, 2224-2228
- **SMAL, M. A. et al.** Activation and cytotoxicity of 2-alpha-aminoacyl prodrugs of methotrexate. *Biochem. Pharmacol.,* 1995, vol. 49, 567-574
- **PRUDENCIO, M. ; RODRIGUEZ, A. ; MOTA, M.M.** The silent path to thousands of merozoites: the Plasmodium liver-stage. *Nat. Rev. Microbiol.,* 2006, vol. 4, 849-856
- **VALECHA, N. et al.** Comparative antirelapse efficacy of CDR1 compound 80/53 (Bulaquine) vs primaquine in double blind clinical trial. *Curr Sci.,* 2001, vol. 80, 561-563
- **WALSH, D. S. et al.** Randomized trial of 3-dose regimens of tafenoquine (WR238605) versus low-dose primaquine for preventing Plasmodium vivax malaria relapse. *Clin Infect Dis.,* 2004, vol. 39, 1095-1103
- **SINGH, A.P. ; SUROLIA, N. ; SUROLIA, A.** Triclosan inhibit the growth of the late liver-stage of Plasmodium. *IUBMB life,* 2009, vol. 61, 923-928
- **SINGH A.P. et al.** Triclosan inhibit the growth of the liver stage of Plasmodium. *IUBMB life,* vol. 61, 923-928